(19) Europäisches Patentamt
European Patent Office
Office européen des brevets

(11) EP 4 578 868 A1

(12) EUROPEAN PATENT APPLICATION
published in accordance with Art. 153(4) EPC

(43) Date of publication:
**02.07.2025 Bulletin 2025/27**

(21) Application number: **23857359.6**

(22) Date of filing: **23.08.2023**

(51) International Patent Classification (IPC):
***C07K 4/00*** *(2006.01)* ***C07K 7/64*** *(2006.01)*
***C12N 15/11*** *(2006.01)*

(52) Cooperative Patent Classification (CPC):
**C07K 4/00; C07K 7/64; C12N 15/11**

(86) International application number:
**PCT/JP2023/030223**

(87) International publication number:
**WO 2024/043250 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.08.2022 JP 2022132147**

(71) Applicant: **FUJIFILM Corporation
Tokyo 106-8620 (JP)**

(72) Inventors:
- **KANEKO, Mai
Ashigarakami-gun, Kanagawa 258-8577 (JP)**
- **TAMURA, Takashi
Ashigarakami-gun, Kanagawa 258-8577 (JP)**
- **SUZUKI, Koo
Ashigarakami-gun, Kanagawa 258-8577 (JP)**
- **TSUMURA, Kyosuke
Ashigarakami-gun, Kanagawa 258-8577 (JP)**
- **OHASHI, Noriyuki
Ashigarakami-gun, Kanagawa 258-8577 (JP)**
- **KOCHI, Masahiro
Ashigarakami-gun, Kanagawa 258-8577 (JP)**
- **HASHIMOTO, Ichihiko
Ashigarakami-gun, Kanagawa 258-8577 (JP)**
- **MIYAHARA, Kenta
Ashigarakami-gun, Kanagawa 258-8577 (JP)**
- **HORIGOME, Hiroki
Ashigarakami-gun, Kanagawa 258-8577 (JP)**

(74) Representative: **Klunker IP
Patentanwälte PartG mbB
Destouchesstraße 68
80796 München (DE)**

Remarks:
The complete document including Reference Table(s) and the Sequence Listing(s) can be downloaded from the EPO website

# (54) CYCLIC PEPTIDE OR SALT THEREOF, AND MDMX INHIBITOR

(57) An object of the present invention is to provide a cyclic peptide or a salt thereof having excellent cell membrane permeability, and an MDMX inhibitor. According to the present invention, there is provided a cyclic peptide or a salt thereof, represented by Formula (1) as defined in the present specification and having the following characteristics (a) to (c).
(a) In a structure of the cyclic peptide, in a case where an axis length in a longest axis direction of a main chain structure is denoted by a, and axis lengths in two other directions which are orthogonal to a and are orthogonal to each other are denoted by b and c, a molecular shape factor r calculated by Formula (2) after a step of carrying out an ellipsoidal approximation for obtaining each of the axis lengths a, b, and c is within a range of 0.4 to 0.6;

$$r = \frac{2\sqrt{b^2 + c^2}}{\sqrt{a^2 + b^2} + \sqrt{c^2 + a^2}} \qquad (2)$$

(b) The main chain structure of the peptide contains a sulfur atom; and
(c) The peptide is non-ionic in a physiological environment.

EP 4 578 868 A1

## Description

Technical Field

The present invention relates to a cyclic peptide having cell membrane permeability, and an MDMX inhibitor.

Background Art

The human transcription factor protein p53 plays an important role in protecting cells from malignant transformation by inducing cellular proliferation arrest or apoptosis in response to DNA damage and cellular stress. MDM2 and MDMX are known as oncoproteins that interact with p53, negatively control the function of p53, and inhibit the transcriptional activity of p53. MDM2 and MDMX are found to be amplified or overexpressed in many cancers and are involved in the development and progression of human cancers. Inhibitors for the p53-MDM2 interaction and the p53-MDMX interaction are capable of restoring the activity of p53 and are therefore expected to be useful as anticancer drugs. In recent years, it has been reported that a low-molecular-weight compound Nutlin-3a or the like, which acts as an MDMX2 inhibitor, efficiently kills cancer cells having a wild-type p53 gene. On the other hand, no effective compound has been reported with regard to the MDMX inhibitor, and no therapeutic method for cancer cells in which MDMX is overexpressed has yet been established.

In recent years, a peptide compound (having a molecular weight of 500 to 2000) has attracted attention because the peptide compound is capable of interacting with a target, which is known as protein-protein interaction (PPI), and may be able to confer cell membrane permeability. In particular, a cyclic peptide has advantages over a linear peptide, such as improved target binding ability to a target protein, specificity, cell membrane permeability, and metabolic stability. For example, a cyclic peptide derived from a naturally occurring product, such as cyclosporin, is marketed as a pharmaceutical product. In order to obtain a high target binding ability with respect to a wide interaction surface of PPI, a cyclic peptide having 10 or more amino acid residues is preferred, but it is known that the cyclic peptide having 10 or more amino acid residues generally does not have high cell membrane permeability.

p53 binds to a hydrophobic pocket of MDMX through binding residues ($Phe^{19}$, $Leu^{22}$, and $Trp^{23}$) in an $\alpha$-helical region of a transactivation (TA) domain of p53. Patent Documents 1 and 2 describe an MDM2/MDMX dual inhibitor, which is a cyclic peptide utilizing staple crosslinking, using the $\alpha$-helical region of p53 as a drug discovery seed. Patent Document 3 and Non-Patent Document 1 describe an MDM2/MDMX dual inhibitor that is a cyclic peptide which mimics the TA domain of p53 by combining $\beta$-turn sequences to form a $\beta$-sheet structure through a cyclosporin-like molecular design. In addition, Patent Document 4 reports a design in which a long-chain alkyl side chain is introduced to improve hydrophobicity and a structural change in a hydrophilic-hydrophobic environment is utilized to confer cell membrane permeability for a thioether cyclic peptide compound.

In addition to the above-mentioned conformationally controlled cyclic peptide, Non-Patent Document 2 describes an MDMX inhibitor that utilizes a low-molecular-weight compound that can bind to the pocket of MDMX instead of the TA domain of p53.

Prior Art Documents

Patent Documents

Patent Document 1: WO2015/153761A
Patent Document 2: WO2013/123266A
Patent Document 3: WO2021/102322A
Patent Document 4: WO2015/030014A

Non-Patent Documents

Non-Patent Document 1: R. Fasan, R. L. A. Dias, K. Moehle, O. Zerbe, D. Obrecht, P. R. E. Mittl, M. G. Grutter, J. A. Robinson. (2006). Structure-Activity Studies in a Family of β-Hairpin Protein Epitope Mimetic Inhibitors of the p53-HDM2 Protein-Protein Interaction. ChemBioChem, 7, 515-526.
Non-Patent Document 2: D. H. Yu, Z. Y. Xu, S. Mo, L. Yuan, X. D. Cheng, J. J. Qin. (2020). Targeting MDMX for Cancer Therapy: Rationale, Strategies, and Challenges. Front. Oncol. 10, article 1389.

## SUMMARY OF THE INVENTION

### Object to be solved by the invention

The cyclic peptide has a high target binding ability to a target, but has low cell membrane permeability. In Patent Document 1, Patent Document 2 and Non-Patent Document 1, attempts are made to improve the cell membrane permeability by a design in which a cyclic peptide is controlled to have a conformation that is favorable for cell membrane permeation. However, problems remain even with any of the attempts, and a new design is still required to confer cell membrane permeability.

As described above, it is considered that the MDMX inhibitor can be an effective therapeutic agent in the treatment of various cancers, and various MDMX inhibitors have been developed, ranging from low-molecular-weight compounds to peptide compounds. However, no inhibitor having sufficient cell membrane permeability has been obtained. An object of the present invention is to provide a cyclic peptide or a salt thereof having excellent cell membrane permeability. Another object of the present invention is to provide an MDMX inhibitor including the above-mentioned cyclic peptide or a salt thereof.

Means for solving the object

As a result of extensive studies to achieve the above-mentioned objects, the present inventors have found that a cyclic peptide or a salt thereof, which has the characteristics (a) to (c) defined in the present specification, has excellent cell membrane permeability. The present invention has been completed based on the above findings. According to the present invention, the following inventions are provided.

<1> A cyclic peptide or a salt thereof, represented by Formula (1) and having the following characteristics (a) to (c).

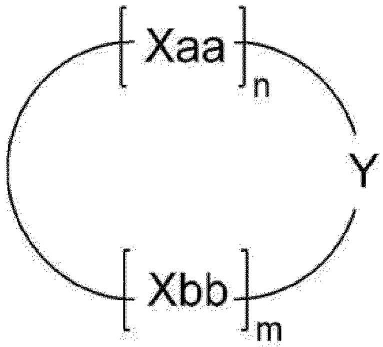

(1)

In the formula, Y represents a divalent group represented by $*\text{-CR}^1\text{-S-CR}^2\text{-}*$, $R^1$ and $R^2$ represent a hydrogen atom or a substituent, * represents a position of bonding to Xaa and Xbb, n pieces of Xaa's each independently represent any amino acid residue or any amino acid analog residue, m pieces of Xbb's each independently represent any amino acid residue or any amino acid analog residue, and n + m represents an integer of 5 to 50;

(a) in a structure of the cyclic peptide, in a case where an axis length in a longest axis direction of a main chain structure is denoted by a, and axis lengths in two other directions which are orthogonal to a and are orthogonal to each other are denoted by b and c, a molecular shape factor r calculated by Formula (2) after a step of carrying out an ellipsoidal approximation for obtaining each of the axis lengths a, b, and c is within a range of 0.4 to 0.6;

$$r = \frac{2\sqrt{b^2 + c^2}}{\sqrt{a^2 + b^2} + \sqrt{c^2 + a^2}} \qquad (2)$$

(b) the main chain structure of the peptide contains a sulfur atom; and
(c) the peptide is non-ionic in a physiological environment.

<2> The cyclic peptide or a salt thereof according to <1>, in which the cyclic peptide is represented by Formula (3).

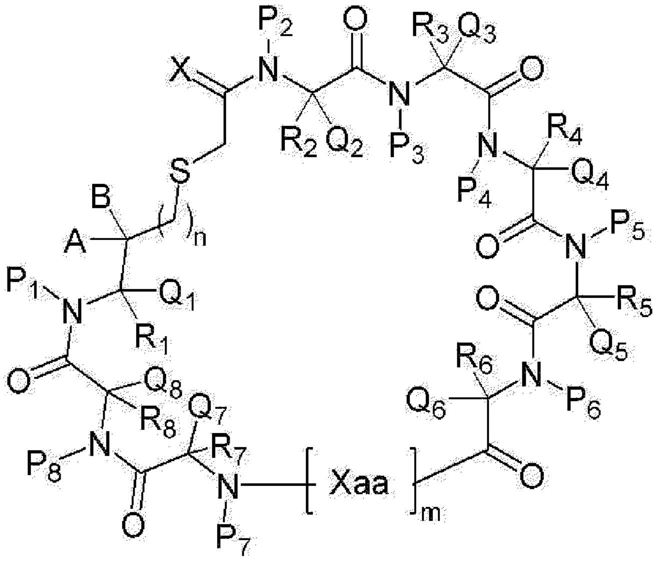

(3)

In the formula, X is an oxygen atom or a sulfur atom, $R_1$, $R_2$, $R_3$, $R_4$, $R_5$, $R_6$, $R_7$, $R_8$, $Q_1$, $Q_2$, $Q_3$, $Q_4$, $Q_5$, $Q_6$, $Q_7$, $Q_8$, A, and B are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, $P_1$, $P_2$, $P_3$, $P_4$, $P_5$, $P_6$, $P_7$, and $P_8$ are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, or a carbon atom to which $R_1$, $R_2$, $R_3$, $R_4$, $R_5$, $R_6$, $R_7$, $R_8$, A, and B are bonded may form a heterocycle together with a nitrogen atom to which $P_1$, $P_2$, $P_3$, $P_4$, $P_5$, $P_6$, $P_7$, and $P_8$ are bonded, m pieces of Xaa's each independently represent any amino acid residue or any amino acid analog residue, and n is an integer of 0 to 5, and m is an integer of 0 to 2.

<3> The cyclic peptide or a salt thereof according to <2>, in which n is 0 or 1.

<4> The cyclic peptide or a salt thereof according to <2>, in which m is 1.

<5> The cyclic peptide or a salt thereof according to <2>, in which at least one of $R_3$ to $R_8$ or $Q_3$ to $Q_8$ is an aromatic carbocyclic group which may have a substituent or an aromatic heterocyclic group which may have a substituent.

<6> The cyclic peptide or a salt thereof according to <2>, in which at least one of $R_3$, $R_5$, $R_6$, $R_7$, $R_8$, $Q_3$, $Q_5$, $Q_6$, $Q_7$, or $Q_8$ is an aromatic carbocyclic group which may have a substituent or an aromatic heterocyclic group which may have a substituent.

<7> The cyclic peptide or a salt thereof according to <2>, in which $R_1$ and $Q_1$ or A and B, and $P_1$ may form a heterocycle together with a carbon atom to which $R_1$ and $Q_1$ or A and B are bonded and a nitrogen atom to which $P_1$ is bonded, a carbon atom to which $R_2$, $R_3$, $R_4$, $R_5$, $R_6$, $R_7$, $R_8$, $Q_2$, $Q_3$, $Q_4$, $Q_5$, $Q_6$, $Q_7$, and $Q_8$ are bonded may form a heterocycle together with a nitrogen atom to which $P_2$, $P_3$, $P_4$, $P_5$, $P_6$, $P_7$, and $P_8$ are bonded, Xaa may be proline, and the cyclic peptide or a salt thereof contains at least one heterocycle by a structure formed from these groups.

<8> The cyclic peptide or a salt thereof according to <2>, in which $R_1$ and $Q_1$ or A and B, and $P_1$ may form a heterocycle together with a carbon atom to which $R_1$ and $Q_1$ or A and B are bonded and a nitrogen atom to which $P_1$ is bonded, a carbon atom to which $R_2$ and $Q_2$ are bonded may form a heterocycle together with a nitrogen atom to which $P_2$ is bonded, Xaa may be proline, and the cyclic peptide or a salt thereof contains at least one heterocycle by a structure formed from these groups.

<9> The cyclic peptide or a salt thereof according to <2>, in which $R_1$ and $Q_1$ or A and B, and $P_1$ may form a pyrrolidine ring together with a carbon atom to which $R_1$ and $Q_1$ or A and B are bonded and a nitrogen atom to which $P_1$ is bonded, a carbon atom to which $R_2$ and $Q_2$ are bonded may form a pyrrolidine ring together with a nitrogen atom to which $P_2$ is bonded, Xaa may be proline, and the cyclic peptide or a salt thereof contains at least one pyrrolidine ring by a structure formed from these groups.

<10> The cyclic peptide or a salt thereof according to <2>, in which $R_1$ and $Q_1$ or A and B and Xaa are each any D-amino acid residue or any D-amino acid analog residue.

<11> An MDMX inhibitor comprising the cyclic peptide or a salt thereof according to any one of <1> to <10>.

<A> A method for inhibiting MDMX, the method comprising administering the cyclic peptide or a salt thereof according to any one of <1> to <10> to a subject.

<B> The cyclic peptide or a salt thereof according to any one of <1> to <10>, in which the cyclic peptide or a salt thereof is used for a treatment of inhibiting MDMX.

<C> Use of the cyclic peptide or a salt thereof according to any one of <1> to <10> for the production of an MDMX inhibitor.

Effect of the invention

The cyclic peptide or a salt thereof and MDMX inhibitor according to the embodiment of the present invention have excellent cell membrane permeability.

## BRIEF DESCRIPTION OF THE DRAWINGS

Fig. 1 shows a three-dimensional structure of a two-dimensionally drawn structural formula of a cyclic peptide.

Fig. 2 shows an ellipsoidal approximation of a structure of a cyclic peptide.

## EMBODIMENTS FOR CARRYING OUT THE INVENTION

Hereinafter, the present invention will be described in more detail.

In the present specification, "to" shows a range including numerical values described before and after "to" as a minimum value and a maximum value, respectively.

<Terminology>

Amino acid refers to a molecule containing both an amino group and a carboxyl group. The amino acid may be any of a natural amino acid or an unnatural amino acid and may be any of D- or L-isomers. The amino acid may be an α-amino acid. The α-amino acid refers to a molecule containing an amino group and a carboxyl group which are bonded to a carbon designated as an α-carbon.

The natural amino acid represents any of alanine (A), arginine (R), asparagine (N), cysteine (C), aspartic acid (D), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y), or valine (V).

The unnatural amino acid refers to an amino acid other than the above-mentioned 20 types of natural amino acids.

The amino acid analog refers to a molecule that is structurally similar to an amino acid and can be used instead of an amino acid in the production of a cyclic peptide.

Examples of the amino acid analog include, but are not particularly limited to, a β-amino acid, and an amino acid in which an amino group or a carboxyl group is similarly substituted with a reactive group (for example, a primary amine is substituted with a secondary or tertiary amine, or a carboxyl group is substituted with an ester). The β-amino acid refers to a molecule containing both an amino group and a carboxyl group in a β configuration.

Examples of the unnatural amino acid residue and the amino acid analog residue include, but are not particularly limited to, the following.

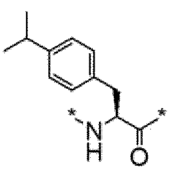
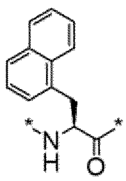
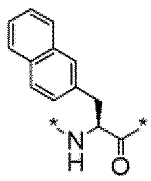
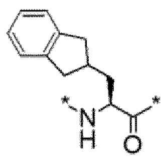
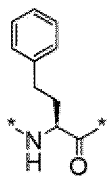

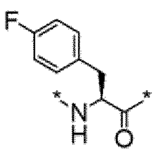
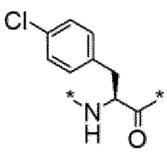
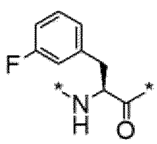
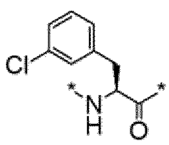
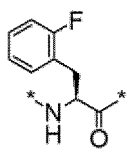

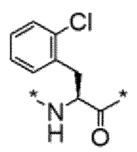 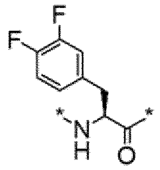 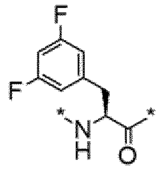 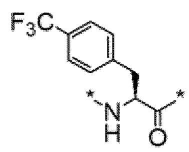 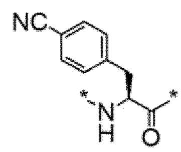

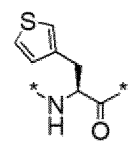 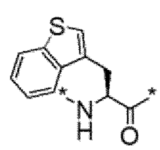 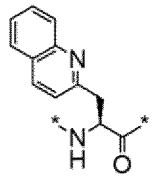 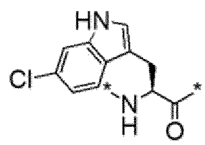 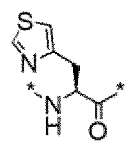

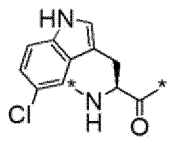 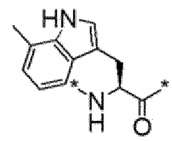 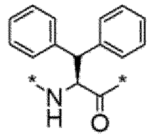 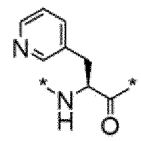 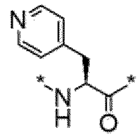

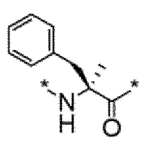 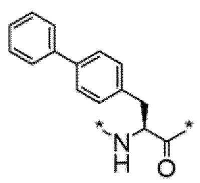 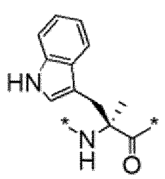 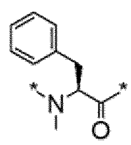 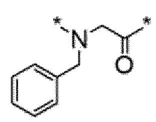

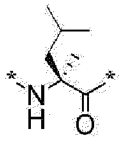 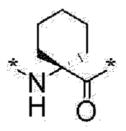 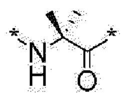 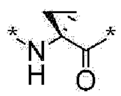 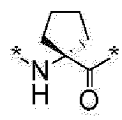

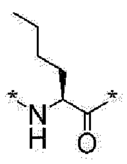 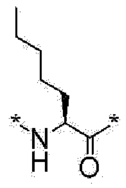 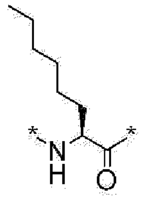 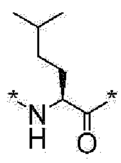 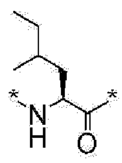

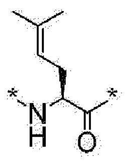 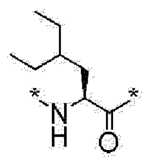 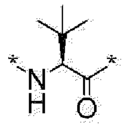 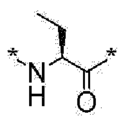 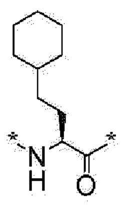

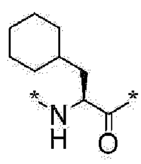 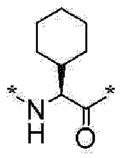 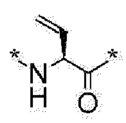 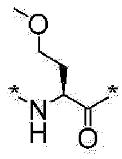 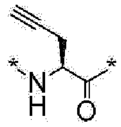

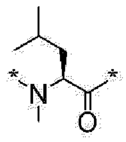 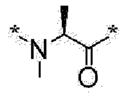 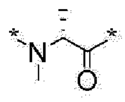 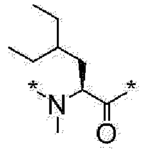 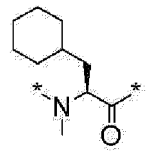

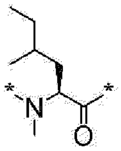 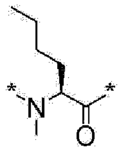 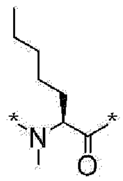 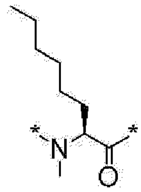 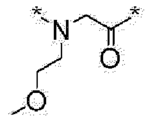 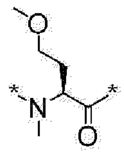

The amino acid analog includes a β-amino acid analog. Examples of the β-amino acid analog include, but are not limited to, the following: cyclic β-amino acid analogs; β-alanine; (R)-β-phenylalanine; (R)-1,2,3,4-tetrahydroisoquinoline-3-acetic acid; (R)-3-amino-4-(1-naphthyl)-butyric acid; (R)-3-amino-4-(2,4-dichlorophenyl)butyric acid; (R)-3-amino-4-(2-chlorophenyl)-butyric acid; (R)-3-amino-4-(2-cyanophenyl)-butyric acid; (R)-3-amino-4-(2-fluorophenyl)-butyric acid; (R)-3-amino-4-(2-furyl)-butyric acid; (R)-3-amino-4-(2-methylphenyl)-butyric acid; (R)-3-amino-4-(2-naphthyl)-butyric acid; (R)-3-amino-4-(2-thienyl)-butyric acid; (R)-3-amino-4-(2-trifluoromethylphenyl)-butyric acid; (R)-3-amino-4-(3,4-dichlorophenyl)butyric acid; (R)-3-amino-4-(3,4-difluorophenyl)butyric acid; (R)-3-amino-4-(3-benzothienyl)-butyric acid; (R)-3-amino-4-(3-chlorophenyl)-butyric acid; (R)-3-amino-4-(3-cyanophenyl)-butyric acid; (R)-3-amino-4-(3-fluorophenyl)-butyric acid; (R)-3-amino-4-(3-methylphenyl)-butyric acid; (R)-3-amino-4-(3-pyridyl)-butyric acid; (R)-3-amino-4-(3-thienyl)-butyric acid; (R)-3-amino-4-(3-trifluoromethylphenyl)-butyric acid; (R)-3-amino-4-(4-bromophenyl)-butyric acid; (R)-3-amino-4-(4-chlorophenyl)-butyric acid;(R)-3-amino-4-(4-cyanophenyl)-butyric acid; (R)-3-amino-4-(4-fluorophenyl)-butyric acid; (R)-3-amino-4-(4-iodophenyl)-butyric acid; (R)-3-amino-4-(4-methylphenyl)-butyric acid; (R)-3-amino-4-(4-nitrophenyl)-butyric acid; (R)-3-amino-4-(4-pyridyl)-butyric acid; (R)-3-amino-4-(4-trifluoromethylphenyl)-butyric acid; (R)-3-amino-4-pentafluoro-phenylbutyric acid; (R)-3-amino-5-hexenoic acid; (R)-3-amino-5-hexynoic acid; (R)-3-amino-5-phenylpentanoic acid; (R)-3-amino-6-phenyl-5-hexenoic acid; (S)-1,2,3,4-tetrahydroisoquinoline-3-acetic acid; (S)-3-amino-4-(1-naphthyl)-butyric acid; (S)-3-amino-4-(2,4-dichlorophenyl)butyric acid; (S)-3-amino-4-(2-chlorophenyl)-butyric acid; (S)-3-amino-4-(2-cyanophenyl)-butyric acid; (S)-3-amino-4-(2-fluorophenyl)-butyric acid; (S)-3-amino-4-(2-furyl)-butyric acid; (S)-3-amino-4-(2-methylphenyl)-butyric acid; (S)-3-amino-4-(2-naphthyl)-butyric acid; (S)-3-amino-4-(2-thienyl)-butyric acid; (S)-3-amino-4-(2-trifluoromethylphenyl)-butyric acid; (S)-3-amino-4-(3,4-dichlorophenyl)butyric acid; (S)-3-amino-4-(3,4-difluorophenyl)butyric acid; (S)-3-amino-4-(3-benzothienyl)-butyric acid; (S)-3-amino-4-(3-chlorophenyl)-butyric acid; (S)-3-amino-4-(3-cyanophenyl)-butyric acid; (S)-3-amino-4-(3-fluorophenyl)-butyric acid; (S)-3-amino-4-(3-methylphenyl)-butyric acid; (S)-3-amino-4-(3-pyridyl)-butyric acid; (S)-3-amino-4-(3-thienyl)-butyric acid; (S)-3-amino-4-(3-trifluoromethylphenyl)-butyric acid; (S)-3-amino-4-(4-bromophenyl)-butyric acid; (S)-3-amino-4-(4-chlorophenyl)-butyric acid; (S)-3-amino-4-(4-cyanophenyl)-butyric acid; (S)-3-amino-4-(4-fluorophenyl)-butyric acid; (S)-3-amino-4-(4-iodophenyl)-butyric acid; (S)-3-amino-4-(4-methylphenyl)-butyric acid; (S)-3-amino-4-(4-nitrophenyl)-butyric acid; (S)-3-amino-4-(4-pyridyl)-butyric acid; (S)-3-amino-4-(4-trifluoromethylphenyl)-butyric acid; (S)-3-amino-4-pentafluorophenylbutyric acid; (S)-3-amino-5-hexenoic acid; (S)-3-amino-5-hexynoic acid; (S)-3-amino-5-phenylpentanoic acid; (S)-3-amino-6-phenyl-5-hexenoic acid; 1,2,5,6-tetrahydropyridine-3-carboxylic acid; 1,2,5,6-tetrahydropyridine-4-carboxylic acid; 3-amino-3-(2-chlorophenyl)-propionic acid; 3-amino-3-(2-thienyl)-propionic acid; 3-amino-3-(3-bromophenyl)-propionic acid; 3-amino-3-(4-chlorophenyl)-propionic acid; 3-amino-3-(4-methoxyphenyl)-propionic acid; 3-amino-4,4,4-trifluoro-butyric acid; 3-amino adipic acid; D-β-phenylalanine; β-leucine; L-β-homoalanine; L-β-homoaspartic acid γ-benzyl ester; L-β-homoglutamic acid δ-benzyl ester; L-β-homoisoleucine; L-β-homoleucine; L-β-homomethionine; L-β-homophenylalanine; L-β-homoproline; L-β-homotryptophan; L-β-homovaline; L-Nω-benzyloxycarbonyl-β-homolysine; Nω-L-β-homoarginine; O-benzyl-L-β-homohydroxyproline; O-benzyl-L-β-homoserine; O-benzyl-L-β-homothreonine; O-benzyl-L-β-homotyrosine; γ-trityl-L-β-homoasparagine; (R)-β-phenylalanine; L-β-homoaspartic acid γ-t-butyl ester; L-β-homoglutamic acid δ-t-butyl ester; L-Nω-β-homolysine; Nδ-trityl-L-β-homoglutamine; Nω-2,2,4,6,7-pentamethyl-dihydrobenzofuran-5-sulfonyl-L-β-homoarginine; O-t-butyl-L-β-homohydroxy-proline; O-t-butyl-L-β-homoserine; O-t-butyl-L-β-homothreonine; O-t-butyl-L-β-homotyrosine; 2-aminocyclopentanecarboxylic acid; and 2-aminocyclohexanecarboxylic acid.

The amino acid analog includes an analog of alanine, valine, glycine, or leucine. Examples of the amino acid analogs of alanine, valine, glycine, and leucine include, but are not limited to, the following: α-methoxyglycine; α-allyl-L-alanine; α-aminoisobutyric acid; α-methyl-leucine; β-(1-naphthyl)-D-alanine; β-(1-naphthyl)-L-alanine; β-(2-naphthyl)-D-alanine; β-(2-naphthyl)-L-alanine; β-(2-pyridyl)-D-alanine; β-(2-pyridyl)-L-alanine; β-(2-thienyl)-D-alanine; β-(2-thienyl)-L-alanine; β-(3-benzothienyl)-D-alanine; β-(3-benzothienyl)-L-alanine; β-(3-pyridyl)-D-alanine; β-(3-pyridyl)-L-alanine; β-(4-pyridyl)-D-alanine; β-(4-pyridyl)-L-alanine; β-chloro-L-alanine; β-cyano-L-alanine; β-cyclohexyl-D-alanine; β-cyclohexyl-L-alanine; β-cyclopenten-1-yl-alanine; β-cyclopentyl-alanine; β-cyclopropyl-L-Ala-OH·dicyclohexylammonium salt; β-t-butyl-D-alanine; β-t-butyl-L-alanine; γ-aminobutyric acid; L-α,β-diaminopropionic acid; 2,4-dinitro-phenylglycine; 2,5-dihydro-D-phenylglycine; 2-amino-4,4,4-trifluorobutyric acid; 2-fluoro-phenylglycine; 3-amino-4,4,4-trifluorobutyric acid; 3-fluoro-valine; 4,4,4-trifluoro-valine; 4,5-dehydro-L-leu-OH·dicyclohexylammonium salt; 4-fluoro-D-phenylglycine; 4-fluoro-L-phenylglycine; 4-hydroxy-D-phenylglycine; 5,5,5-trifluoro-leucine; 6-aminohexanoic acid; cyclopentyl-D-Gly-OH·dicyclohexylammonium salt; cyclopentyl-Gly-OH·dicyclohexylammonium salt; D-α,β-diaminopropionic acid; D-α-aminobutyric acid; D-α-t-butylglycine; D-(2-thienyl)glycine; D-(3-thienyl)glycine; D-2-aminocaproic acid; D-2-indanyl glycine; D-allylglycine·dicyclohexylammonium salt; D-cyclohexylglycine; D-norvaline; D-phenylglycine; β-aminobutyric acid; β-aminoisobutyric acid; (2-bromophenyl)glycine; (2-methoxyphenyl)glycine; (2-methylphenyl)glycine; (2-thiazoyl)glycine; (2-thienyl)glycine; 2-amino-3-(dimethylamino)-propionic acid; L-α,β-diaminopropionic acid; L-α-aminobutyric acid; L-α-t-butylglycine; L-(3-thienyl)glycine; L-2-amino-3-(dimethylamino)-propionic acid; L-2-aminocaproic acid dicyclohexyl-ammonium salt; L-2-indanyl glycine; L-allylglycine·dicyclohexylammonium salt; L-cyclohexylglycine; L-phenylglycine; L-propargylglycine; L-norvaline; N-α-aminomethyl-L-alanine; D-α,γ-diaminobutyric acid; L-α,γ-diaminobutyric acid; β-cyclopropyl-L-alanine; (N-β-(2,4-dinitrophenyl))-L-α,β-diaminopropionic acid; (N-β-1-(4,4-dimethyl-2,6-dioxocyclohexa-1-ylidene)ethyl)-D-α,β-diaminopropionic acid; (N-β-1-(4,4-dimethyl-2,6-dioxocyclohexa-1-ylidene)ethyl)-L-α,β-diaminopropionic acid; (N-β-4-methyltrityl)-L-α,β-diaminopropionic acid; (N-β-allyloxycarbonyl)-L-α,β-diaminopropionic acid; (N-γ-1-(4,4-dimethyl-2,6-dioxocyclohexa-1-ylidene)ethyl)-D-α,γ-diaminobutyric acid; (N-γ-1-(4,4-dimethyl-2,6-dioxocyclohexa-1-ylidene)ethyl)-L-α,γ-diaminobutyric acid; (N-γ-4-methyltrityl)-D-α,γ-diaminobutyric acid; (N-γ-4-methyltrityl)-L-α,γ-diaminobutyric acid; (N-γ-allyloxycarbonyl)-L-α,γ-diaminobutyric acid; D-α,γ-diaminobutyric acid; 4,5-dehydro-L-leucine; cyclopentyl-D-Gly-OH; cyclopentyl-Gly-OH; D-allylglycine; D-homocyclohexylalanine; L-1-pyrenylalanine; L-2-aminocaproic acid; L-allylglycine; L-homocyclohexylalanine; and N-(2-hydroxy-4-methoxy-Bzl)-Gly-OH.

The amino acid analog includes analogs of phenylalanine and tyrosine. Examples of the amino acid analogs of phenylalanine and tyrosine include the following: β-methyl-phenylalanine, β-hydroxyphenylalanine, α-methyl-3-methoxy-DL-phenylalanine, α-methyl-D-phenylalanine, α-methyl-L-phenylalanine, 1,2,3,4-tetrahydroisoquinoline-3-carboxylic acid, 2,4-dichloro-phenylalanine, 2-(trifluoromethyl)-D-phenylalanine, 2-(trifluoromethyl)-L-phenylalanine, 2-bromo-D-phenylalanine, 2-bromo-L-phenylalanine, 2-chloro-D-phenylalanine, 2-chloro-L-phenylalanine, 2-cyano-D-phenylalanine, 2-cyano-L-phenylalanine, 2-fluoro-D-phenylalanine, 2-fluoro-L-phenylalanine, 2-methyl-D-phenylalanine, 2-methyl-L-phenylalanine, 2-nitro-D-phenylalanine, 2-nitro-L-phenylalanine, 2,4,5-trihydroxy-phenylalanine, 3,4,5-trifluoro-D-phenylalanine, 3,4,5-trifluoro-L-phenylalanine, 3,4-dichloro-D-phenylalanine, 3,4-dichloro-L-phenylalanine, 3,4-difluoro-D-phenylalanine, 3,4-difluoro-L-phenylalanine, 3,4-dihydroxy-L-phenylalanine, 3,4-dimethoxy-L-phenylalanine,3,5,3'-triiodo-L-thyronine, 3,5-diiodo-D-tyrosine, 3,5-diiodo-L-tyrosine, 3,5-diiodo-L-thyronine, 3-(trifluoromethyl)-D-phenylalanine, 3-(trifluoromethyl)-L-phenylalanine, 3-amino-L-tyrosine, 3-bromo-D-phenylalanine, 3-bromo-L-phenylalanine, 3-chloro-D-phenylalanine, 3-chloro-L-phenylalanine, 3-chloro-L-tyrosine, 3-cyano-D-phenylalanine, 3-cyano-L-phenylalanine, 3-fluoro-D-phenylalanine, 3-fluoro-L-phenylalanine, 3-fluoro-tyrosine, 3-iodo-D-phenylalanine, 3-iodo-L-phenylalanine, 3-iodo-L-tyrosine, 3-methoxy-L-tyrosine, 3-methyl-D-phenylalanine, 3-methyl-L-phenylalanine, 3-nitro-D-phenylalanine,3-nitro-L-phenylalanine, 3-nitro-L-tyrosine, 4-(trifluoromethyl)-D-phenylalanine, 4-(trifluoromethyl)-L-phenylalanine, 4-amino-D-phenylalanine, 4-amino-L-phenylalanine, 4-benzoyl-D-phenylalanine, 4-benzoyl-L-phenylalanine, 4-bis(2-chloroethyl)amino-L-phenylalanine, 4-bromo-D-phenylalanine, 4-bromo-L-phenylalanine, 4-chloro-D-phenylalanine, 4-chloro-L-phenylalanine, 4-cyano-D-phenylalanine, 4-cyano-L-phenylalanine, 4-fluoro-D-phenylalanine, 4-fluoro-L-phenylalanine, 4-iodo-D-phenylalanine, 4-iodo-L-phenylalanine, homophenylalanine, thyroxine, 3,3-diphenylalanine, thyronine, ethyl-tyrosine, and methyl-tyrosine.

The amino acid analog includes an analog of proline. Examples of the amino acid analog of proline include, but are not limited to, the following: 3,4-dehydro-proline, 4-fluoro-proline, cis-4-hydroxy-proline, thiazolidine-2-carboxylic acid, and trans-4-fluoro-proline.

The amino acid analog includes analogs of serine and threonine. Examples of the amino acid analogs of serine and threonine include, but are not limited to, the following: 3-amino-2-hydroxy-5-methylhexanoic acid, 2-amino-3-hydroxy-4-methylpentanoic acid, 2-amino-3-ethoxybutanoic acid, 2-amino-3-methoxybutanoic acid, 4-amino-3-hydroxy-6-methylheptanoic acid, 2-amino-3-benzyloxypropionic acid, 2-amino-3-benzyloxypropionic acid, 2-amino-3-ethoxypropionic acid, 4-amino-3-hydroxybutanoic acid, and α-methylserine.

The amino acid analog includes an analog of tryptophan. Examples of the amino acid analog of tryptophan include, but are not limited to, the following: α-methyl-tryptophan; β-(3-benzothienyl)-D-alanine; β-(3-benzothienyl)-L-alanine; 1-methyl-tryptophan; 4-methyl-tryptophan; 5-benzyloxy-tryptophan; 5-bromo-tryptophan; 5-chloro-tryptophan; 5-fluoro-tryptophan; 5-hydroxy-tryptophan; 5-hydroxy-L-tryptophan; 5-methoxy-tryptophan; 5-methoxy-L-tryptophan; 5-methyl-tryptophan; 6-bromo-tryptophan; 6-chloro-D-tryptophan; 6-chloro-tryptophan; 6-fluoro-tryptophan; 6-methyl-tryptophan; 7-benzyloxy-tryptophan; 7-bromo-tryptophan; 7-methyl-tryptophan; D-1,2,3,4-tetrahydro-norharmane-3-carboxylic acid; 6-methoxy-1,2,3,4-tetrahydro-norharmane-1-carboxylic acid; 7-azatryptophan; L-1,2,3,4-tetrahydro-norharmane-3-carboxylic acid; 5-methoxy-2-methyl-tryptophan; and 6-chloro-L-tryptophan.

In one example, the amino acid analog is racemic. Either the D-isomer of the amino acid analog may be used, or the L-isomer of the amino acid analog may be used. In addition, the amino acid analog may contain a chiral center in the R or S configuration. Further, the amino group (singular or plural) of the β-amino acid analog may be substituted with a protective group such as tert-butyloxycarbonyl (BOC group), 9-fluorenylmethyloxycarbonyl (FMOC), or tosyl. Further, the carboxylic acid functional group of the β-amino acid analog may be protected, for example, as an ester derivative thereof. In addition, a salt of the amino acid analog may be used.

The alkyl group includes a linear or branched hydrocarbon group having 1 to 15 carbon atoms, preferably 1 to 10 carbon atoms, and more preferably 1 to 6 carbon atoms. Examples of the alkyl group include, but are not limited to, methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl. In addition, at least one carbon atom in these alkyl groups may be substituted with a heteroatom, such as oxygen, nitrogen, sulfur, phosphorus, or silicon. The alkenyl group includes a linear or branched hydrocarbon group having 2 to 15 carbon atoms, preferably 2 to 10 carbon atoms, and more preferably 2 to 6 carbon atoms, which has one or more carbon-carbon double bonds at any position. Examples of the alkenyl group include, but are not limited to, vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl. In addition, at least one carbon atom in these alkenyl groups may be substituted with a heteroatom. The alkynyl group includes a linear or branched hydrocarbon group having 2 to 10 carbon atoms, preferably 2 to 8 carbon atoms, and more preferably 2 to 6 carbon atoms, which has one or more carbon-carbon triple bonds at any position. Further, the alkynyl group may have a double bond at any position. Examples of the alkynyl group include, but are not limited to, ethynyl, propynyl, butynyl, pentynyl, hexynyl, heptynyl, octynyl, nonynyl, and decynyl. In addition, at least one carbon atom in these alkynyl groups may be substituted with a heteroatom.

The aromatic carbocyclic group is a carbocyclic group containing a single aromatic ring or a plurality of aromatic rings. The number of carbon atoms in the aromatic carbocyclic group is preferably 5 to 24, more preferably 5 to 18, and still more preferably 5 to 14. Examples of the aromatic carbocyclic group include phenyl and naphthyl.

The non-aromatic carbocyclic group is a carbocyclic group containing a single non-aromatic ring or a plurality of non-aromatic rings. The number of carbon atoms in the non-aromatic carbocyclic group is preferably 3 to 24, more preferably 3 to 18, and still more preferably 3 to 14. Examples of the non-aromatic carbocyclic group include saturated and unsaturated cyclic hydrocarbon groups, for example, cyclopropyl, cyclobutyl, cyclopentyl, cyclopentenyl, cyclohexyl, cyclohexenyl, cycloheptyl, and cyclooctyl.

The aromatic heterocyclic group refers to an aromatic 5- to 8-membered monocyclic, 8- to 12-membered bicyclic, or 11- to 14-membered tricyclic ring system having 1 to 3 heteroatoms in a monocyclic ring system, 1 to 6 heteroatoms in a bicyclic ring system, or 1 to 9 heteroatoms in a tricyclic ring system. The heteroatom can be selected from O, N, or S. Examples of the aromatic heterocyclic group include, but are not limited to, a pyridyl group, a pyrimidinyl group, a pyrrolyl group, a furyl group, a furanyl group, a thiophenyl group, a thienyl group, an indolyl group, an isoindolyl group, an indolidinyl group, an imidazolyl group, a pyridonyl group, a pyrimidyl group, a pyrazinyl group, an oxazolyl group, a thiazolyl group, a purinyl group, a quinolinyl group, an isoquinolinyl group, a benzimidazolyl group, a benzofuranyl group, and a benzoxazolyl group.

The non-aromatic heterocyclic group refers to a non-aromatic 5- to 8-membered monocyclic, 8- to 12-membered bicyclic, or 11- to 14-membered tricyclic ring system having 1 to 3 heteroatoms in a monocyclic ring system, 1 to 6 heteroatoms in a bicyclic ring system, or 1 to 9 heteroatoms in a tricyclic ring system. The heteroatom can be selected from O, N, or S. Examples of the non-aromatic heterocyclic group include, but are not limited to, piperazinyl, pyrrolidinyl, dioxanyl, morpholinyl, and tetrahydrofuranyl.

The heterocycle includes the above-mentioned aromatic heterocyclic group and non-aromatic heterocyclic group.

The phrase "may have a substituent" indicates that, in any of the above-mentioned chemical groups (an alkyl group, an alkenyl group, an alkynyl group, an aromatic carbocyclic group, a non-aromatic carbocyclic group, an aromatic heterocyclic group, and a non-aromatic heterocyclic group), one or more hydrogen atoms are substituted with an atom or chemical group other than a hydrogen atom. Examples of the substituent include, but are not particularly limited to, halogen (fluorine, chlorine, bromine, iodine, or the like), hydroxy, mercapto, oxo, nitro, haloalkyl having 1 to 10 carbon atoms, alkyl having 1 to 10 carbon atoms, aryl having 6 to 20 carbon atoms, aralkyl having 7 to 20 carbon atoms, alkoxy having 1 to 10 carbon atoms, thioalkoxy having 1 to 10 carbon atoms, aryloxy having 6 to 20 carbon atoms, amino, alkoxycarbonyl having 2 to 10 carbon atoms, amide, carboxy, alkanesulfonyl having 1 to 10 carbon atoms, alkylcarbonyl having 2 to 10 carbon atoms, and a cyano group.

$R^1$ and $R^2$ in the divalent group represented by $*\text{-CR}^1\text{-S-CR}^2\text{-}*$ may each represent a substituent, and examples of the substituent include, but are not particularly limited to, halogen (fluorine, chlorine, bromine, iodine, or the like), hydroxy, mercapto, oxo, nitro, haloalkyl having 1 to 10 carbon atoms, alkyl having 1 to 10 carbon atoms, aryl having 6 to 20 carbon atoms, aralkyl having 7 to 20 carbon atoms, alkoxy having 1 to 10 carbon atoms, thioalkoxy having 1 to 10 carbon

atoms, aryloxy having 6 to 20 carbon atoms, amino, alkoxycarbonyl having 2 to 10 carbon atoms, amide, carboxy, alkanesulfonyl having 1 to 10 carbon atoms, alkylcarbonyl having 2 to 10 carbon atoms, and a cyano group.

<Cyclic peptide>

The cyclic peptide according to the embodiment of the present invention is represented by Formula (1) and has the following characteristics (a) to (c).

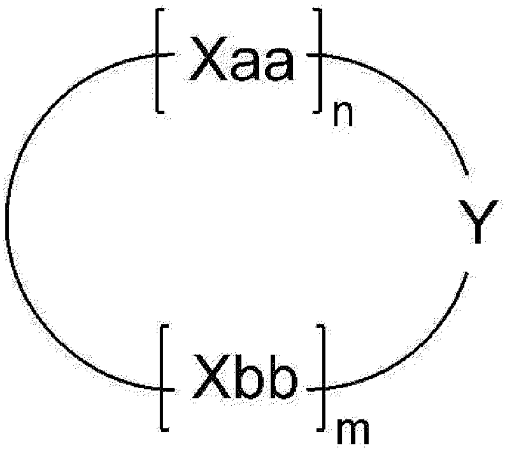

(1)

In the formula, Y represents a divalent group represented by *-C$R^1$-S-C$R^2$-*, $R^1$ and $R^2$ represent a hydrogen atom or a substituent, * represents a position of bonding to Xaa and Xbb, n pieces of Xaa's each independently represent any amino acid residue or any amino acid **analog** residue, m pieces of Xbb's each independently represent any amino acid residue or any amino acid analog residue, and n + m represents an integer of 5 to 50.

n + m preferably represents an integer of 5 to 50, more preferably an integer of 5 to 20, and still more preferably an integer of 9 to 11.

The cyclic peptide according to the embodiment of the present invention has high intracellular permeability. The cyclic peptide according to the embodiment of the present invention is capable of inhibiting MDMX-p53 interaction and is therefore useful in the treatment of cancers, such as solid tumors, in which MDMX is overexpressed.

(a) In a structure of the cyclic peptide, in a case where an axis length in a longest axis direction of a main chain structure is denoted by a, and axis lengths in two other directions which are orthogonal to a and are orthogonal to each other are denoted by b and c, a molecular shape factor r calculated by Formula (2) after a step of carrying out an ellipsoidal approximation (see Fig. 2) for obtaining each of the axis lengths a, b, and c is within a range of 0.4 to 0.6.

$$r = \frac{2\sqrt{b^2 + c^2}}{\sqrt{a^2 + b^2} + \sqrt{c^2 + a^2}} \qquad (2)$$

The molecular shape factor r calculated by Expression (2) is preferably 0.4 to 0.6 and more preferably 0.4 to 0.55.

(b) The main chain structure of the peptide contains a sulfur atom.

(c) The peptide is non-ionic in a physiological environment. By non-ionic in a physiological environment is meant that the peptide does not have a substituent having a charge in a physiological environment.

The cyclic peptide according to the embodiment of the present invention is preferably represented by Formula (3).

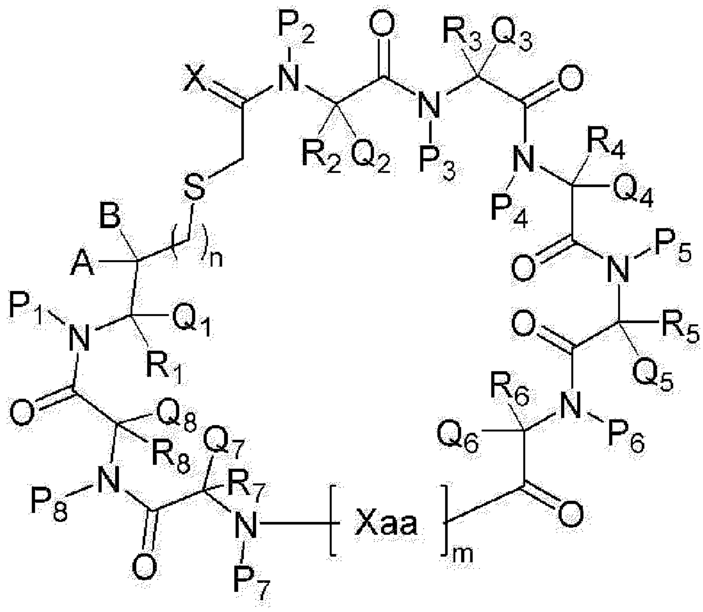

(3)

In the formula, X is an oxygen atom or a sulfur atom, $R_1$, $R_2$, $R_3$, $R_4$, $R_5$, $R_6$, $R_7$, $R_8$, $Q_1$, $Q_2$, $Q_3$, $Q_4$, $Q_5$, $Q_6$, $Q_7$, $Q_8$, A, and B are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, $P_1$, $P_2$, $P_3$, $P_4$, $P_5$, $P_6$, $P_7$, and $P_8$ are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, or a carbon atom to which $R_1$, $R_2$, $R_3$, $R_4$, $R_5$, $R_6$, $R_7$, $R_8$, A, and B are bonded may form a heterocycle together with a nitrogen atom to which $P_1$, $P_2$, $P_3$, $P_4$, $P_5$, $P_6$, $P_7$, and $P_8$ are bonded, m pieces of Xaa's each independently represent any amino acid residue or any amino acid analog residue, and n is an integer of 0 to 5, and m is an integer of 0 to 2.

Preferably, n is 0 or 1.

Preferably, m is 1.

Preferably, at least one of $R_3$ to $R_8$ or $Q_3$ to $Q_8$ is an aromatic carbocyclic group which may have a substituent or an aromatic heterocyclic group which may have a substituent.

Preferably, at least one of $R_3$, $R_5$, $R_6$, $R_7$, or $R_8$ or $Q_3$, $Q_5$, $Q_6$, $Q_7$, or $Q_8$ is an aromatic carbocyclic group which may have a substituent or an aromatic heterocyclic group which may have a substituent.

Preferably, $R_1$ and $Q_1$ or A and B, and $P_1$ may form a heterocycle together with a carbon atom to which $R_1$ and $Q_1$ or A and B are bonded and a nitrogen atom to which $P_1$ is bonded, and a carbon atom to which $R_2$, $R_3$, $R_4$, $R_5$, $R_6$, $R_7$, $R_8$, $Q_2$, $Q_3$, $Q_4$, $Q_5$, $Q_6$, $Q_7$, and $Q_8$ are bonded may form a heterocycle together with a nitrogen atom to which $P_2$, $P_3$, $P_4$, $P_5$, $P_6$, $P_7$, and $P_8$ are bonded, and Xaa may be proline, and the cyclic peptide contains at least one heterocycle by a structure formed from these groups.

Preferably, $R_1$ and $Q_1$ or A and B, and $P_1$ may form a heterocycle together with a carbon atom to which $R_1$ and $Q_1$ or A and B are bonded and a nitrogen atom to which $P_1$ is bonded, a carbon atom to which $R_2$ and $Q_2$ are bonded may form a heterocycle together with a nitrogen atom to which $P_2$ is bonded, Xaa may be proline, and the cyclic peptide contains at least one heterocycle by a structure formed from these groups.

Preferably, $R_1$ and $Q_1$ or A and B, and $P_1$ may form a pyrrolidine ring together with a carbon atom to which $R_1$ and $Q_1$ or A and B are bonded and a nitrogen atom to which $P_1$ is bonded, a carbon atom to which $R_2$ and $Q_2$ are bonded may form a pyrrolidine ring together with a nitrogen atom to which $P_2$ is bonded, Xaa may be proline, and the cyclic peptide contains at least one pyrrolidine ring by a structure formed from these groups.

Preferably, $R_1$ and $Q_1$ or A and B and Xaa are each any D-amino acid residue or any D-amino acid analog residue.

The cyclic peptide according to the embodiment of the present invention may be modified by phosphorylation, methylation, acetylation, adenylylation, ADP-ribosylation, glycosylation, or the like, depending on the use application. In addition, the cyclic peptide according to the embodiment of the present invention may be a salt. The salts are preferably

salts with physiologically acceptable inorganic and organic acids and bases. Examples of appropriate acid salts include the following: acetate, adipate, benzoate, benzenesulfonate, butyrate, citrate, digluconate, dodecyl sulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, pamoate, phosphate, picrate, pivalate, propionate, salicylate, succinate, sulfate, tartrate, tosylate, and undecanoate. Salts derived from appropriate bases include an alkali metal (for example, sodium) salt, an alkaline earth metal (for example, magnesium) salt, an ammonium salt, and an N-(alkyl)$^{4+}$ salt.

<Method for producing cyclic peptide>

The method for producing the cyclic peptide according to the embodiment of the present invention is not particularly limited. The cyclic peptide according to the embodiment of the present invention may be produced by a method using a cell-free translation system, or may be produced by a chemical synthesis method of a peptide. The chemical synthesis of a peptide can generally be carried out using an automated peptide synthesizer.

The peptide may be synthesized by either a solid phase synthesis method or a liquid phase synthesis method, among which a solid phase synthesis method is preferable. The solid phase synthesis of a peptide is known to those skilled in the art, and involves, for example, an esterification reaction between a hydroxyl group of a resin having a hydroxyl group and a carboxyl group of a first amino acid (usually a C-terminal amino acid of a desired peptide) in which an α-amino group is protected with a protective group. A known dehydration condensation agent such as 1-mesitylenesulfonyl-3-nitro-1,2,4-triazole (MSNT), dicyclohexylcarbodiimide (DCC), or diisopropylcarbodiimide (DIPCDI) can be used as an esterification catalyst. Next, the protective group of the α-amino group of the first amino acid is eliminated, a second amino acid in which all functional groups of a main chain except a carboxy group are protected is added, and the carboxy group is activated to bond the first amino acid and the second amino acid. Further, the α-amino group of the second amino acid is deprotected, a third amino acid in which all functional groups of a main chain except a carboxy group are protected is added, and the carboxy group is activated to bond the second amino acid and the third amino acid. This process is repeated and in a case where a peptide having a desired length is synthesized, all functional groups are deprotected. Examples of resins for solid phase synthesis include a Merrifield resin, an MBHA resin, a Cl-Trt resin, a SASRIN resin, a Wang resin, a Rink amide resin, an HMFS resin, an Amino-PEGA resin (Merck), and an HMPA-PEGA resin (Merck). These resins may be washed with a solvent (dimethylformamide (DMF), 2-propanol, methylene chloride, or the like) before use. Examples of the protective group for the α-amino group include a benzyloxycarbonyl (Cbz or Z) group, a tert-butoxycarbonyl (Boc) group, a fluorenylmethoxycarbonyl (Fmoc) group, a benzyl group, an allyl group, and an allyloxycarbonyl (Alloc) group. The Cbz group can be deprotected by hydrofluoric acid, hydrogenation, or the like, the Boc group can be deprotected by trifluoroacetic acid (TFA), and the Fmoc group can be deprotected by a treatment with piperidine. For protection of an α-carboxy group, a methyl ester, an ethyl ester, a benzyl ester, a tert-butyl ester, a cyclohexyl ester, or the like can be used. As for other functional groups of amino acids, a hydroxyl group of serine or threonine can be protected with a benzyl group or a tert-butyl group, and a hydroxyl group of tyrosine can be protected with a 2-bromobenzyloxycarbonyl group or a tert-butyl group. An amino group in a side chain of lysine and a carboxy group of glutamic acid or aspartic acid can be protected in the same manner as the α-amino group and the α-carboxy group. The activation of the carboxy group can be carried out using a condensing agent. Examples of the condensing agent include dicyclohexylcarbodiimide (DCC), diisopropylcarbodiimide (DIPCDI), 1-ethyl-3-(3-dimethylaminopropyl) carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU). Cleavage of a peptide chain from the resin can be carried out by a treatment with an acid such as TFA or hydrogen fluoride (HF).

The method for thioether cyclization of a peptide is not particularly limited. For example, the peptide can be cyclized by including the following functional groups in a side chain or main chain of the peptide. The positions of functional groups 1 and 2 are not particularly limited, and either of functional groups 1 and 2 may be located at the N-terminal and C-terminal of the peptide, both of functional groups 1 and 2 may be located at the terminals, one of functional groups 1 and 2 may be terminal and the other of functional groups 1 and 2 may be non-terminal, or both of functional groups 1 and 2 may be non-terminal.

The synthesis step and the cyclization reaction step of the peptide compound may be separate or may proceed consecutively. The cyclization can be carried out by methods known to those skilled in the art, for example, methods described in WO2013/100132, WO2008/117833, WO2012/074129, and the like.

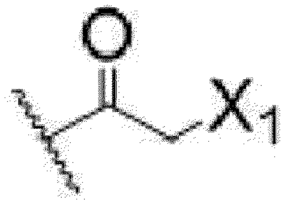

Functional group 1

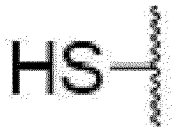

Functional group 2

In the formula, $X_1$ represents chlorine, bromine, or iodine.

<MDMX inhibitor>

According to the present invention, an MDMX inhibitor containing the cyclic peptide or a salt thereof according to the embodiment of the present invention is provided.

As shown in Examples, the cyclic peptide or a salt thereof according to the embodiment of the present invention has an MDMX inhibitory effect. The "MDMX inhibitory effect" refers to the effect of inhibiting the binding of MDMX to p53.

The gene sequence and amino acid sequence of human MDMX are registered at the National Center for Biotechnology Information (NCBI) under Gene ID:4194.

The cyclic peptide according to the embodiment of the present invention can be used in a pharmaceutical composition for the treatment of a disease in which an MDMX inhibitor is involved. The administration form of the pharmaceutical composition is not particularly limited and may be oral administration or parenteral administration. Examples of the parenteral administration include injection such as intramuscular injection, intravenous injection, or subcutaneous injection, transdermal administration, and transmucosal administration (pernasal, buccal, intraocular, transpulmonary, transvaginal, or transrectal). The cyclic peptide in the pharmaceutical composition may be subjected to various modifications in consideration of the properties of being easily metabolized and excreted. For example, polyethylene glycol (PEG) or a sugar chain can be added to a cyclic polypeptide to increase its retention time in blood and reduce its antigenicity. In addition, using a biodegradable polymer compound such as polylactic acid, polyglycolic acid, or PLGA, a porous hydroxyapatite, a liposome, a surface-modified liposome, an emulsion adjusted with an unsaturated fatty acid, a nanoparticle, a nanosphere, or the like as a sustained release base, the cyclic peptide may be encapsulated therein. In a case of transdermal administration, a weak electric current can also be passed through the skin surface to penetrate the stratum corneum.

The pharmaceutical composition may be formulated by using an active ingredient as it is or by adding a carrier, an excipient, an additive, or the like, which is pharmaceutically acceptable. Examples of dosage forms of the pharmaceutical composition include a liquid preparation (for example, an injection), a dispersion, a suspension, a tablet, a pill, a powdered preparation, a suppository, a powder, a fine granule, a granule, a capsule, a syrup, a troche, an inhalant, an ointment, an eye drop, a nasal drop, an ear drop, and a poultice.

The formulation can be carried out by a conventional method using, for example, an excipient, a binder, a disintegrating agent, a lubricant, a solubilizer, a solubilizing aid, a coloring agent, a taste masking agent, a stabilizer, an emulsifier, an absorption enhancer, a surfactant, a pH adjuster, a preservative, and an antioxidant as appropriate.

Examples of ingredients used for formulation include, but are not limited to, purified water, saline, a phosphate buffer solution, dextrose, glycerol, a pharmaceutically acceptable organic solvent such as ethanol, animal and vegetable oils, lactose, mannitol, glucose, sorbitol, crystalline cellulose, hydroxypropyl cellulose, starch, corn starch, anhydrous silicic acid, magnesium aluminum silicate, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, and human serum albumin. As the absorption enhancer that improves the absorption of the cyclic peptide, surfactants such as polyoxyethylene lauryl ethers, sodium lauryl sulfate, and saponin; bile salts such as glycocholic acid, deoxycholic acid, and taurocholic acid; chelating agents such as EDTA and salicylic acids; fatty acids such as caproic acid, capric acid, lauric acid, oleic acid, linoleic acid, and mixed micelles; enamine derivatives, N-acyl collagen peptides, N-acyl amino acids, cyclodextrins, chitosans, nitric oxide donors, and the like may be used. The pill or tablet can also be coated with a sugar coating, a gastric-soluble substance, or an enteric-soluble substance. The injection can contain distilled water for injection, saline, propylene glycol, polyethylene glycol, vegetable oil, alcohols, and the like. Further, a wetting agent, an emulsifier, a dispersing agent, a stabilizer, a solubilizer, a solubilizing aid, a preservative, and the like can be added.

<Use applications of cyclic peptide and MDMX inhibitor>

The cyclic peptide and MDMX inhibitor according to the embodiment of the present invention can be used as a pharmaceutical product, a cosmetic product, a drug delivery system (DDS) material, and the like, without being limited thereto.

The cyclic peptide and the MDMX inhibitor according to the embodiment of the present invention are useful in a competitive binding assay to identify a medicinal agent that binds to MDMX. For example, in a p53/MDMX system, a labeled cyclic peptide according to the embodiment of the present invention can be used in an MDMX binding assay together with a small molecule that competitively binds to MDMX. The competitive binding test allows for rapid in vitro evaluation and determination of drug candidates specific to the p53/MDMX system. Such a binding test can be carried out using the cyclic peptide according to the embodiment of the present invention.

The cyclic peptide according to the embodiment of the present invention can also be used for the production of an antibody against the cyclic peptide according to the embodiment of the present invention.

The cyclic peptide and MDMX inhibitor according to the embodiment of the present invention can be used for the treatment of a subject having a disorder associated with abnormal (for example, insufficient or excessive) expression or activity of p53 or MDMX.

The disorder is caused by an abnormal level (for example, overexpression or underexpression) of p53 or MDMX or by the presence of p53 or MDMX exhibiting an abnormal activity.

The cyclic peptide and MDMX inhibitor according to the embodiment of the present invention can be used for the treatment or prevention of diseases such as hyperproliferative diseases and inflammatory disorders by interfering with the interaction or binding between p53 and MDMX.

**In** the above, an effective amount of the cyclic peptide or MDMX inhibitor according to the embodiment of the present invention can be administered to a mammal, including a human.

As an example, the cyclic peptide and MDMX inhibitor according to the embodiment of the present invention can be used for the treatment, prevention, and/or diagnosis of cancers and neoplastic diseases. In the present specification, cancer, hyperproliferative, and neoplastic refer to cells that have the capacity for autonomous proliferation; that is, an abnormal condition or disease characterized by rapidly proliferating cell proliferation.

Examples of the cancers or neoplastic diseases include, but are not limited to, the following: fibrosarcoma, myosarcoma, liposarcoma, chondrosarcoma, osteogenic sarcoma, chordoma, angiosarcoma, endothelial sarcoma, lymphangiosarcoma, lymphangioendothelial sarcoma, synovialoma, mesothelioma, Ewing's tumor, leiomyosarcoma, rhabdomyosarcoma, gastric cancer, esophageal cancer, rectal cancer, pancreatic cancer, ovarian cancer, prostate cancer, uterine cancer, head and neck cancer, skin cancer, brain cancer, squamous cell cancer, sebaceous gland cancer, breast cancer (including papillary carcinoma, papillary adenocarcinoma, and the like), cystadenocarcinoma, medullary cancer, bronchogenic carcinoma, renal cell cancer, hepatocellular cancer, cholangiocarcinoma, choriocarcinoma, seminoma, embryonic cell cancer, Wilms tumor, cervical cancer, testicular cancer, lung cancer (including small cell lung cancer, non-small cell lung cancer, and the like), bladder cancer, epithelial cancer, glioma, astrocytoma, medulloblastoma, craniopharyngioma, ependymoma, pinealoma, angioblastoma, acoustic neuroma, oligodendroglioma, meningioma, melanoma, neuroblastoma, retinoblastoma, leukemia, lymphoma, and Kaposi's sarcoma.

Examples of the proliferative disorder include a hematopoietic neoplastic disorder. The hematopoietic neoplastic disorder includes a disease involving hyperplastic/neoplastic cells derived from the hematopoietic system, for example originating from the myeloid, lymphoid or erythroid lineages, or precursor cells thereof. The disease can result from acute undifferentiated leukemias, for example, erythroblastic leukemia and acute megakaryoblastic leukemia.

The present invention will be described with reference to the following examples, but the present invention is not limited thereto.

## Examples

Unless otherwise specified, purification by column chromatography was carried out using an automated purification device ISOLERA (manufactured by Biotage AB). Unless otherwise specified, SNAP KP-Sil Cartridge (manufactured by Biotage AB) was used as a carrier in silica gel column chromatography.

The mixing ratio in an eluent is based on a volume ratio. For example, "chloroform/methanol = 90/10 $\rightarrow$ 50/50" means that an eluent of "chloroform/methanol = 90/10" was changed to an eluent of "chloroform/methanol = 50/50".

Mass spectrum (MS) was measured using an ACQUITY SQD LC/MS System (manufactured by Waters Corporation, ionization method: electrospray ionization (ESI) method).

Retention time (RT) was measured using an ACQUITY SQD LC/MS System (manufactured by Waters Corporation) and shown in minutes (min).

Column: BEH C18, 1.7 $\mu$m, 2.1 x 30 mm, manufactured by Waters Corporation

Solvent: liquid A: 0.1% formic acid-water
liquid B: 0.1% formic acid-acetonitrile
Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: room temperature
Detection wavelength: 254 nm
MS means MS (ESI m/z):(M+H), unless otherwise specified.

Nuclear magnetic resonance (NMR) spectra were measured using Bruker AV300 (manufactured by Bruker Corporation) with tetramethylsilane as an internal standard, and all δ values were expressed in ppm.

<Example 1>

The synthesis route of the cyclic peptide according to the embodiment of the present invention is shown. The cyclic peptide according to the embodiment of the present invention can be produced, for example, by a general synthetic method shown below. $R_1$, $Q_1$, A, and B in Formula (4) are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, and n represents an integer of 0 to 5. In addition, PG represents a protective group, examples of which include a fluorenylmethoxycarbonyl (Fmoc) group and a tert-butoxycarbonyl (Boc) group.

General method for synthesis of thioether cyclic peptide

Step 1 Supporting of thiol group-containing compound of Formula (4) on 2-chlorotrityl chloride resin

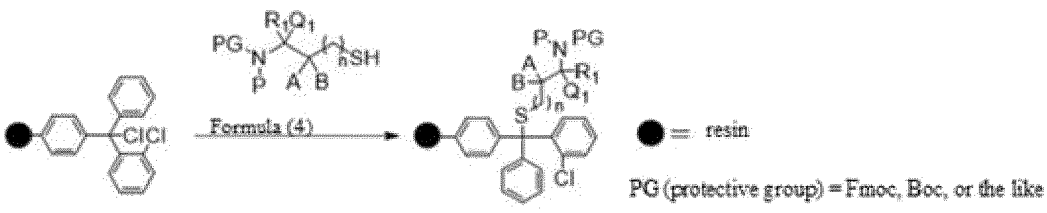


Step 2 Solid phase synthesis of peptide by automated peptide synthesizer

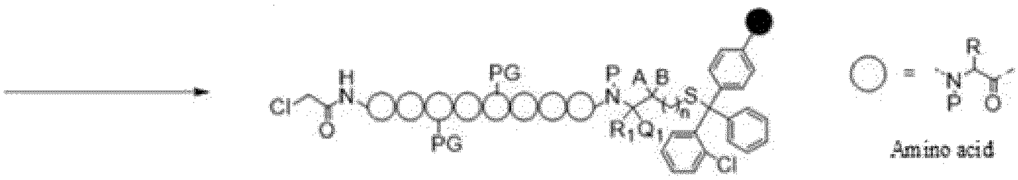


Step 3 Cleavage deprotection

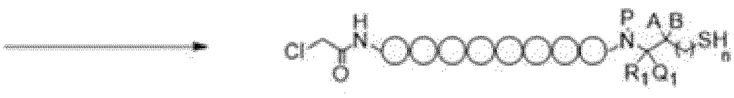

Step 4 Cyclization/Step 5 Purification

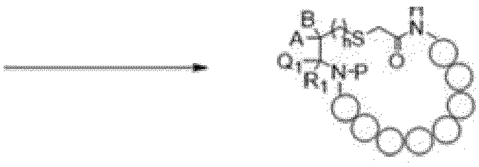

Step 1: Supporting of thiol group-containing compound on 2-chlorotrityl chloride resin

2-Chlorotrityl chloride (100 to 200 mesh, 1% DVB, purchased from Watanabe Chemical Industries, Ltd., 100 mg, 0.137 mmol) and dehydrated dichloromethane (2 mL) were placed in a reaction container (10 mL) equipped with a filter, which was then shaken at room temperature for 10 minutes. After removing dichloromethane by filtration, a mixed liquid obtained by adding a dichloromethane (0.5 M) solution of 4 molar equivalents of a thiol group-containing compound represented by Formula (3) and 5 molar equivalents of diisopropylethylamine to the resin was added to the reaction container which was then shaken for 2 hours. After removing the reaction liquid by filtration, a mixed liquid obtained by adding dichloromethane methanol: diisopropylethylamine = 17:2:1 (2 mL) was added to the reaction container which was then shaken for 15 minutes. After removing the reaction liquid by filtration, the same operation was repeated twice. After removing the reaction liquid by filtration, the resin was washed with dimethylformamide (2 mL) and dichloromethane (2 mL) three or more times each. The resin was dried overnight under reduced pressure to obtain an Fmoc-amino acid-2-chlorotrityl resin. The loading rate of the obtained resin was calculated from the absorbance after Fmoc deprotection.

Step 2: Solid phase synthesis of peptide by automated peptide synthesizer

Solid phase synthesis of a peptide was carried out using an automated peptide synthesizer (Syro I, manufactured by Biotage AB). Detailed procedures of the operation were carried out in accordance with the manual attached to the peptide synthesizer. The synthesis was carried out by setting a resin for solid phase synthesis (0.05 mmol), an N-methyl-2-pyrrolidone (NMP) solution of Fmoc amino acid (0.5 M), an NMP solution of chloroacetic acid (0.5 M), an NMP solution of 1-[bis(dimethylamino)methylene]-1H-1,2,3-triazolo[4,5-b]pyridinium 3-oxide hexafluorophosphate (0.5 M), an NMP solution of 1-hydroxybenzotriazole (0.5 M), an NMP solution of diisopropylethylamine (0.1 M), and an NMP solution of piperidine (20 %v/v) in the peptide synthesizer. A cycle consisting of Fmoc deprotection (20 minutes), washing with NMP, condensation of Fmoc amino acids (1 hour), and washing with NMP as one cycle was repeated to elongate the peptide chain. After condensation of an amino acid at the N-terminal, chloroacetic acid was condensed to the N-terminal amino group in the same manner.

Step 3: Cleavage from resin and deprotection of protective group of side chain functional group

In order to simultaneously carry out the cleavage of a linear peptide from the resin and the deprotection of the protective group of the side chain functional group, a solution of trifluoroacetic acid:triisopropylsilane:dichloromethane = 5:2.5:92.5 corresponding to 5 times the amount of the resin was added to the resin, followed by shaking at room temperature for 2 hours. The reaction liquid was recovered by filtration. The reaction was further repeated once using a solution of trifluoroacetic acid:triisopropylsilane:dichloromethane having the same concentration, and the reaction liquid was recovered by filtration. The recovered reaction liquids were all combined, the solvent was distilled off under reduced pressure, and the residue was thoroughly dried to obtain a crude purified product of a linear peptide.

Step 4: Thioether cyclization reaction

The crude purified product of the linear peptide was dissolved in a solution of acetonitrile (10 mL) and 0.1 M TEAB buffer:pure water = 1:9 (10 mL), and the solution was adjusted to a pH of 8.5 ± 0.1. 1 molar equivalent of a TCEP solution (0.5 M) was added thereto, followed by stirring at room temperature for 30 minutes to 48 hours. After confirming the disappearance of the linear peptide as the raw material by LC/MS mass spectrometry (Acquity UPLC/SQD, manufactured by Waters Corporation), the solvent was distilled off under reduced pressure to obtain a crude purified product of a cyclic peptide.

Step 5: Purification of cyclic peptide

Purification of the obtained crude purified product was carried out by liquid chromatography. Finally, a desired cyclic peptide was obtained as a freeze-dried powder.

Column: X Select CSH Prep C18 5 μm OBD (19 x 250 mm), manufactured by Waters Corporation
Column temperature: 40°C
Flow rate: 20 ml/min
Detection wavelength: 220 nm, 254 nm
Solvent: liquid A: 0.1% formic acid-water
liquid B: 0.1% formic acid-acetonitrile

N-methyl-2-pyrrolidone, diisopropylethylamine, 1-[bis(dimethylamino)methylene]-1H-1,2,3-triazolo[4,5-b]pyridinium 3-oxide hexafluorophosphate, 1-hydroxybenzotriazole, piperidine, and chloroacetic acid were obtained from FUJIFILM Wako Pure Chemical Corporation.

The following Fmoc-amino acids were used in the synthesis of the cyclic peptides described in the present specification. In addition, the following abbreviations are used in the sequences set forth in the present specification. The Fmoc-amino acids were purchased from Watanabe Chemical Industries, Ltd., CHEM-IMPEX International Inc., Acrotein ChemBio Inc., or Amatek Chemical Co., Ltd.

Ala: alanine
Met: methionine
Gly: glycine
Tyr: tyrosine
Trp: tryptophan
Gln: glutamine
Thr: threonine
Phe: phenylalanine
Nle: norleucine
Phe(4-F): 4-F phenylalanine
Ala(4-Thz): 4-thiazolylalanine
HSe(Me): O-methyl-homoserine
Tle: tert-leucine
Cha: cyclohexylalanine
Ahp(2): 2-aminoheptanoic acid
Aoc(2): 2-aminobutyric acid
Phe(4-CF3): 4-trifluoromethylphenylalanine
Phe(4-Cl): 4-chlorophenylalanine
Phe(4-CN): 4-cyanophenylalanine
Phe(3,4-F2): 3,4-difluorophenylalanine
Ala(4-Pyri): 4-pyridylalanine
MeAla: N-methylalanine
MeNle: N-methylnorleucine
MeHSe(Me): N-methyl-O-methyl-homoserine
MeCha: N-methylcyclohexylalanine
MeLeu: N-methylleucine
MePhe: N-methylphenylalanine
D-Pro: D-proline
D-MeAla: D-N-methylalanine

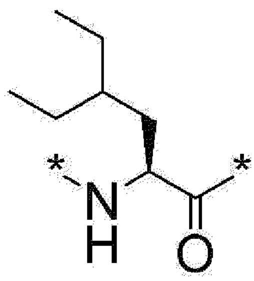

4-EtNle: 4-ethyl-norleucine

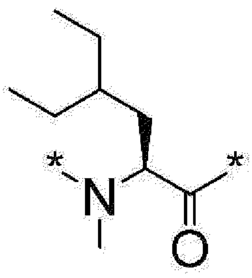

Me4-EtNle: N-methyl-4-ethyl-norleucine

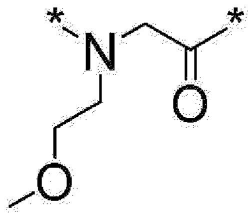

N(2-MeOEt)Gly: N-(2-methoxyethyl)-glycine

Synthesis of Fmoc amino acid analogs having thiol side chains for use in peptide synthesis

Synthesis of N-((9H-fluoren-9-yl)methyl(2-mercaptoethyl))carbamate (compound aa3, Fmoc-C2SH)

N-((9H-fluoren-9-yl)methyl(2-mercaptoethyl))carbamate (Compound aa3, Fmoc-C2SH) was synthesized according to the following route.

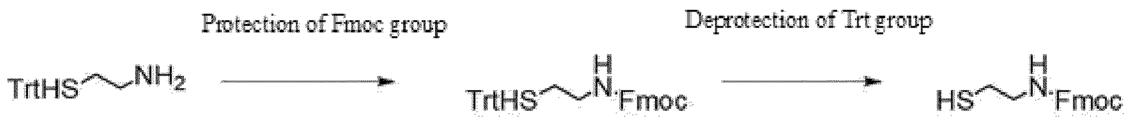


Compound aa1 Compound aa2 Compound aa3

Synthesis of N-((9H-fluoren-9-yl)methyl(2-(trityl-sulfanyl)ethyl)carbamate (compound aa2)

Dimethylformamide (10 mL/g, 10 mL) was added to commercially available 2-(tritylthio)ethanamine (Compound aa1, available from Combi-Blocks, Inc.) (1 g, 3.13 mmol), and the mixture was stirred using a mechanical stirrer. Then, diisopropylethylamine (0.66 mL, 3.76 mmol) and Fmoc N-hydroxysuccinimide ester (1.16 g, 3.44 mmol) were added thereto at room temperature. The reaction liquid was stirred at room temperature for 2 hours. Pure water (10 mL) was added to the reaction liquid at room temperature, followed by washing twice with a 25% ethyl acetate/hexane solution. The resulting aqueous layer was adjusted to a pH of 7 with saturated aqueous citric acid. This was followed by extraction twice with ethyl acetate, and the resulting organic layer was collected and washed with saturated saline. The resulting organic layer was dried over anhydrous sodium sulfate, filtered, and distilled off under reduced pressure to obtain a crude purified product. The crude purified product was purified by column chromatography (silica gel, ethyl acetate/hexane = 0/100 → 20/80) to obtain a title compound aa2 (1.02 g, yield: 60%) as a pale yellow oil.

MS (ESI m/z): (M+H) 542.7
RT (min): 2.24

Synthesis of N-((9H-fluoren-9-yl)methyl(2-mercaptoethyl))carbamate (compound aa3, Fmoc-C2SH)

A solution (10 mL) of trifluoroacetic acid:3,6-dioxa-1,8-octanedithiol:triisopropylsilane:dichloromethane = 88:5:2:5 was added to the compound aa2 (1 g, 1.85 mmol) at room temperature, followed by stirring for 4 hours using a mechanical stirrer. Toluene (5 mL) was added to the reaction liquid, and the solvent was distilled off under reduced pressure. Pure water (10 mL) was added to the residue which was then adjusted to a pH of 7 with a 1 M NaOH aqueous

solution. This was followed by extraction twice with ethyl acetate, and the resulting organic layer was collected and washed with saturated saline. The resulting organic layer was dried over anhydrous sodium sulfate, filtered, and distilled off under reduced pressure to obtain a crude purified product. The crude purified product was purified by column chromatography (silica gel, ethyl acetate/hexane = 0/100 → 20/80) to obtain a title compound aa3 (0.54 g, yield: 98%) as a white solid.

MS (ESI m/z): (M+H) 300.1
RT (min): 1.57
1H-NMR ($CDCl_3$) δ: 7.78-7.76 (2H, m), 7.60-7.58 (2H, m), 7.43-7.39 (2H, m), 7.34-7.30 (2H, m), 5.25 (1H, brs), 4.44-4.42 (2H, m), 4.24-4.20 (1H, m), 3.40-3.35 (2H, m), 2.69-2.63 (2H, m), 1.36-1.32 (1H, m).

Synthesis of N-((9H-fluoren-9-yl)methylfS)-(1-mercapto-3-(thiazol-4-yl)propan-2-yl)carbamate (compound aa8, Fmoc-Ala(4-Thz)-SH)

N-((9H-fluoren-9-yl)methyl(S)-(1-mercapto-3-(thiazol-4-yl)propan-2-yl)carbamate (compound aa8, Fmoc-Ala(4-Thz)-SH) was synthesized according to the following route.

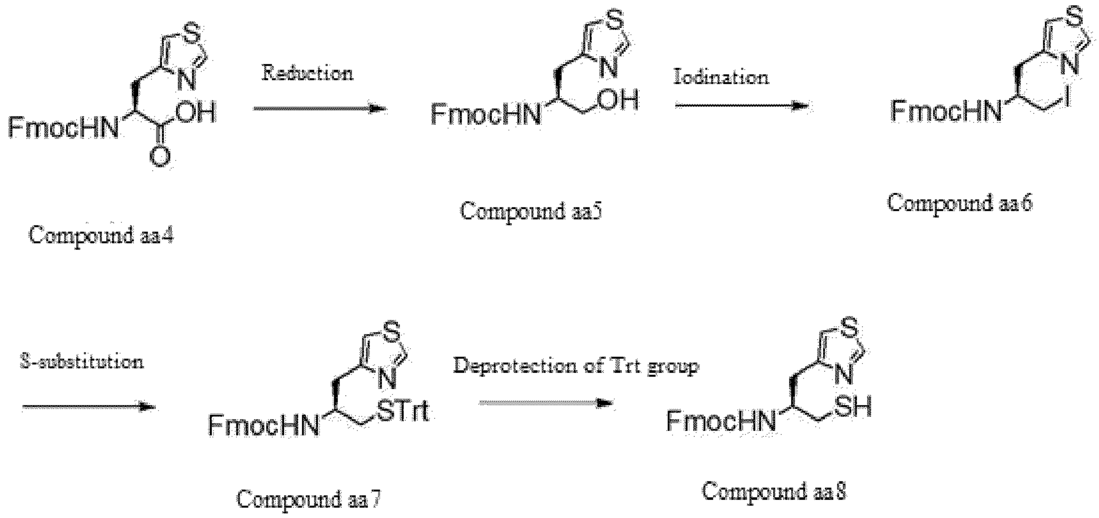


Compound aa4
Compound aa5
Compound aa6
Compound aa7
Compound aa8

Synthesis of N-((9H-fluoren-9-yl)methyl(S)-(1-hydroxy-3-(thiazol-4-yl)propan-2-yl)carbamate (compound aa5)

Under a nitrogen atmosphere, tetrahydrofuran (8 mL/g, 8 mL) and 4-methylmorpholine (0.31 mL, 2.79 mmol) were added to commercially available Fmoc-Ala(4-Thz)-OH (1 g, 2.54 mmol, available from Watanabe Chemical Industries, Ltd.), and the mixture was stirred using a mechanical stirrer. The reaction liquid was cooled to -30°C or lower using a methanol-dry ice bath, and isobutyl chloroformate (0.36 mL, 2.79 mmol) was added dropwise thereto. The reaction liquid was stirred at -30°C for 30 minutes, and then an aqueous solution (3 M, 3 mL, 0.28 g) of oily sodium hydride (60%) was added dropwise thereto over 5 minutes or more. The reaction liquid was stirred at -30°C for 30 minutes. Thereafter, using an ice bath, saturated aqueous citric acid (20 mL) was slowly added dropwise to the reaction liquid which was then stirred for 30 minutes. The temperature was raised to room temperature, followed by extraction twice with ethyl acetate (20 mL), and the resulting organic layer was collected and washed with saturated saline. The resulting organic layer was dried over anhydrous sodium sulfate, filtered, and distilled off under reduced pressure to obtain a crude purified product. The crude purified product was purified by column chromatography (silica gel, ethyl acetate/methanol = 100/0 → 0/100) to obtain a title compound aa5 (0.68 g, yield: 70%) as a white solid.

MS (ESI m/z): (M+H) 381.1
RT (min): 1.33

Synthesis of N-((9H-fluoren-9-yl)methyl(S)-(1-iodo-3-(thiazol-4-yl)propan-2-yl)carbamate (compound aa6)

The compound aa5 (0.68 g, 1.78 mmol), triphenylphosphine (0.93 g, 3.56 mmol), and iodine (0.89 g, 3.56 mmol) were dissolved in tetrahydrofuran (8 mL/g, 5.4 mL), and imidazole (0.29 g, 4.27 mmol) was slowly added thereto over 5 minutes or more on an ice bath. After the completion of addition, the temperature of the solution was raised to room temperature, followed by stirring for 22 hours. Pure water (10 mL) was added to the reaction liquid, followed by extraction

twice with ethyl acetate (10 mL), and the resulting organic layer was collected and washed with saturated saline. The resulting organic layer was dried over anhydrous magnesium sulfate, filtered, and distilled off under reduced pressure to obtain a crude purified product. The crude purified product was purified by column chromatography (silica gel, ethyl acetate/hexane = 0/100 → 30/70) to obtain a title compound aa6 (0.28 g, yield: 22%) as a colorless liquid.

MS (ESI m/z): (M+H) 491.2
RT (min): 1.74

Synthesis of N-((9H-fluoren-9-yl)methyl(S)-(1-(thiazol-4-yl)-3-(tritylthio)propan-2-yl)carbamate (compound aa7)

Under a nitrogen atmosphere, oily sodium hydride (60%) (0.041 g, 1.0 mmol) was dissolved in dimethylformamide (10 mL/g, 2 mL), and triphenylmethanethiol (0.29 g, 1.06 mmol) was added thereto, followed by stirring at room temperature for 1 hour. A dimethylformamide solution (1 mL/g, 0.2 mL) of the compound aa6 (0.20 g, 0.41 mmol) was added dropwise to the reaction liquid which was then stirred at room temperature for 1 hour. Then, pure water (10 mL) was slowly added dropwise thereto, followed by stirring for 30 minutes. This was followed by extraction twice with ethyl acetate (10 mL), and the resulting organic layer was collected and washed with saturated saline. The resulting organic layer was dried over anhydrous sodium sulfate, filtered, and distilled off under reduced pressure. The resulting residue was dissolved in dimethylformamide (10 mL/g, 2 mL), and diisopropylethylamine (0.09 mL, 0.49 mmol) and Fmoc N-hydroxysuccinimide ester (0.15 g, 0.45 mmol) were added thereto at room temperature. After stirring for 3 hours, pure water (10 mL) was added to the reaction liquid at room temperature, followed by washing twice with a 25% ethyl acetate/hexane solution. The resulting aqueous layer was adjusted to a pH of 7 with saturated aqueous citric acid. This was followed by extraction twice with ethyl acetate, and the resulting organic layer was collected and washed with saturated saline. The resulting organic layer was dried over anhydrous sodium sulfate, filtered, and distilled off under reduced pressure to obtain a crude purified product. The crude purified product was purified by column chromatography (silica gel, ethyl acetate/hexane = 0/100 → 10/90) to obtain a title compound aa7 (0.20 g, yield: 79%) as a white oil.

MS (ESI m/z): (M+H) 639.1
RT (min): 2.21

Synthesis of N-((9H-fluoren-9-yl)methyl(S)-(1-mercapto-3-(thiazol-4-yl)propan-2-yl)carbamate (compound aa8, Fmoc-Ala(4-Thz)-SH)

A solution (7 mL) of trifluoroacetic acid:triisopropylsilane:dichloromethane = 25:5:70 was added to the compound aa7 (0.21 g, 0.32 mmol) at room temperature, followed by stirring for 30 minutes using a mechanical stirrer. Toluene (5 mL) was added to the reaction liquid, and the solvent was distilled off under reduced pressure. Pure water (10 mL) was added to the residue which was then adjusted to a pH of 7 with a 1 M NaOH aqueous solution. This was followed by extraction twice with ethyl acetate, and the resulting organic layer was collected and washed with saturated saline. The resulting organic layer was dried over anhydrous sodium sulfate, filtered, and distilled off under reduced pressure to obtain a crude purified product. The crude purified product was purified by column chromatography (silica gel, ethyl acetate/methanol = 100/0 → 0/100) to obtain a title compound aa8 (0.13 g, yield: > 99%) as a white solid.

MS (ESI m/z): (M+H) 397.1
RT (min): 1.61
1H-NMR ($CDCl_3$) δ: 8.82 (1H, s), 7.77-7.50 (2H, m), 7.61-7.53 (2H, m), 7.44-7.36 (2H, m), 7.34-7.27 (2H, m), 7.10 (1H, brs), 5.76-5.74 (1H, m), 4.76 (1H, brs), 4.39-4.37 (2H, m), 4.21-4.14 (1H, m), 3.29-3.08 (2H, m), 2.81-2.67 (1H, m), 2.65-2.47 (1H, m), 1.52-1.39 (1H, m).

Synthesis of N-((9H-fluoren-9-yl)methyl(R)-2-(mercaptomethyl)pyrrolidine-1-carboxylate (compound aa13, Fmoc-D-Pro-SH)

N-((9H-fluoren-9-yl)methyl(R)-2-(mercaptomethyl)pyrrolidine-1-carboxylate (compound aa13, Fmoc-D-Pro-SH) was synthesized according to the following route.

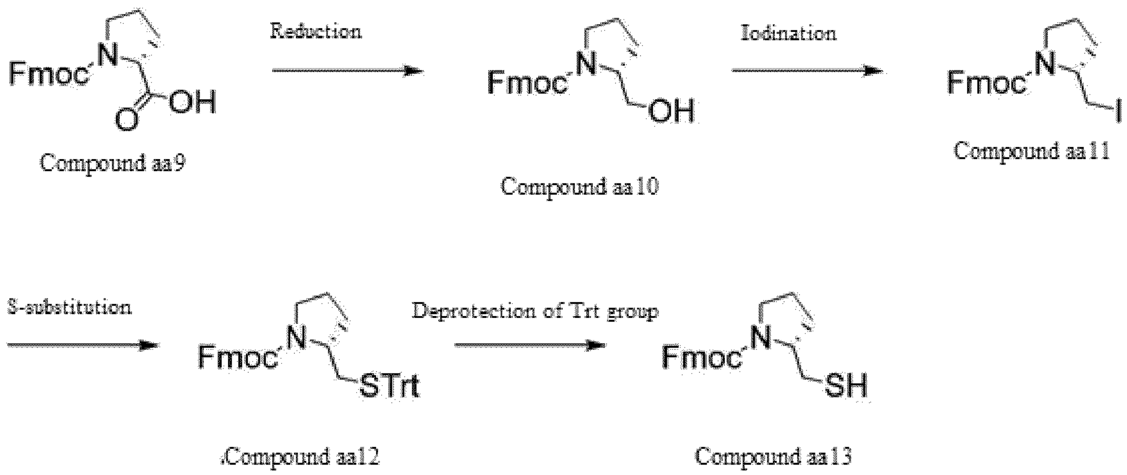


Compound aa9
Compound aa10
Compound aa11
Compound aa12
Compound aa13

Synthesis of N-((9H-fluoren-9-yl)methyl(R)-2-(hydroxymethyl)pyrrolidine-1-carboxylate (compound aa10)

The synthesis was carried out in the same manner as in the synthesis of N-((9H-fluoren-9-yl)methyl(S)-(1-hydroxy-3-(thiazol-4-yl)propan-2-yl)carbamate (compound aa5) from commercially available Fmoc-D-Pro-OH (compound aa9, available from Watanabe Chemical Industries, Ltd.). The compound aa10 was used without purification in the synthesis of the compound aa11.

Synthesis of N-((9H-fluoren-9-yl)methyl(R)-2-(iodomethyl)pyrrolidine-1-carboxylate (compound aa11)

The synthesis was carried out in the same manner as in the synthesis of N-((9H-fluoren-9-yl)methyl(S)-(1-iodo-3-(thiazol-4-yl)propan-2-yl)carbamate (compound aa6).

MS (ESI m/z): (M+H) 434.0
RT (min): 1.96

Synthesis of N-((9H-fluoren-9-yl)methyl(R)-2-((tritylthio)methyl)pyrrolidine-1-carboxylate (compound aa12)
The synthesis was carried out in the same manner as in the synthesis of N-((9H-fluoren-9-yl)methyl(S)-(1-(thiazol-4-yl)-3-(tritylthio)propan-2-yl)carbamate (compound aa7).
1H-NMR ($CDCl_3$) $\delta$: 7.81-7.72 (2H, m), 7.61-7.54 (2H, m), 7.48-7.10 (19H, m), 4.39-4.27 (1H, m), 4.25-4.10 (2H, m), 4.00-3.85 (1H, m), 3.43-3.27 (2H, m), 2.73-2.59 (1H, m), 2.31-2.15 (1H, m), 1.97-1.57 (4H, m).
Synthesis of N-((9H-fluoren-9-yl)methyl(R)-2-(mercaptomethyl)pyrrolidine-1-carboxylate (compound aa13, Fmoc-D-Pro-SH)
The synthesis was carried out in the same manner as in the synthesis of N-((9H-fluoren-9-yl)methyl(S)-(1-mercapto-3-(thiazol-4-yl)propan-2-yl)carbamate (compound aa 8, Fmoc-Ala(4-Thz)-SH).

MS (ESI m/z): (M+H) 340.1
RT (min): 1.82

Synthesis of cyclic peptide 1

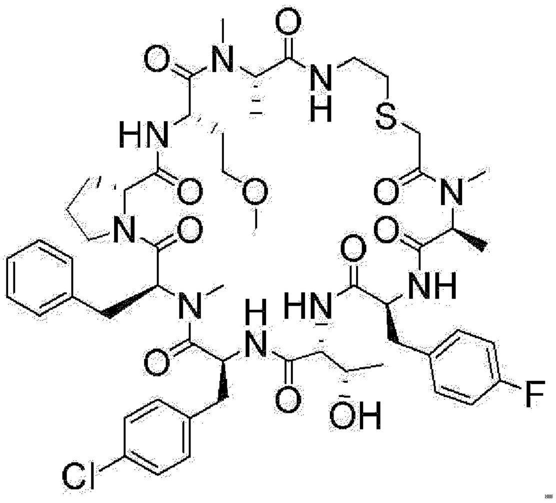

According to Step 1 of the general method, the thiol group of the compound aa3 was supported on a 2-chlorotrityl chloride resin. An Fmoc-C2S-2-chlorotrityl resin (1.01 mmol/g, 0.05 mmol, 50 mg) was suspended in NMP, which was then allowed to swell for 1 hour, and the NMP was removed by filtration. Condensation was carried out in the order of Fmoc-NMe-Ala, Fmoc-Hse(Me)-OH, Fmoc-D-Pro-OH, Fmoc-NMe-Phe-OH, Fmoc-Phe(4-Cl)-OH, Fmoc-Thr(Trt)-OH, Fmoc-Phe(4-F)-OH, Fmoc-NMe-Ala-OH, and chloroacetic acid. After the completion of elongation, the resin was washed with NMP and dichloromethane and then the solvent was distilled off under reduced pressure. TFA:triisopropylsilane:dichloromethane = 5:2.5:92.5 (2 mL) was added thereto, and cleavage and deprotection of the peptide were carried out. After 2 hours, the resin was filtered out and the filtrate was recovered. Further, TFA:triisopropylsilane:dichloromethane = 5:2.5:92.5 (1 mL) was added twice, followed by washing, and the filtrate was recovered. The recovered filtrates were all combined, the solvent was distilled off under reduced pressure, and the residue was thoroughly dried to obtain a crude purified product of a linear peptide. The crude purified product thus obtained was dissolved in a solution of acetonitrile (10 mL) and 0.1 M TEAB buffer:pure water = 1:9 (10 mL), and the solution was adjusted to a pH of 8.5 ± 0.1. 1 molar equivalent of a TCEP solution (0.5 M) was added thereto, followed by stirring at room temperature for 2 hours. Thereafter, the solvent was distilled off under reduced pressure to obtain a crude purified product of a cyclic peptide. The crude purified product thus obtained was dissolved in a small amount of acetonitrile and purified by reverse phase HPLC (0.1% formic acid aqueous solution/0.1% formic acid-acetonitrile solution) to obtain a cyclic peptide 1 (5.99 mg, yield: 10.8%) as a freeze-dried powder.

MS (ESI m/z): (M+H) 1108.5
RT (min): 1.54

Synthesis of cyclic peptide 2

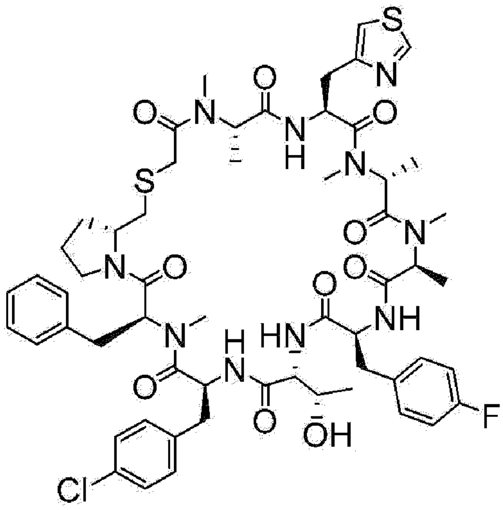

According to Step 1 of the general method, the thiol group of the compound aa13 was supported on a 2-chlorotrityl chloride resin. An Fmoc-D-Pro-S-2-chlorotrityl resin (0.99 mmol/g, 0.05 mmol, 51 mg) was suspended in NMP, which was then allowed to swell for 1 hour, and the NMP was removed by filtration. Condensation was carried out in the order of Fmoc-NMe-Phe-OH, Fmoc-Phe(4-Cl)-OH, Fmoc-Thr(Trt)-OH, Fmoc-Phe(4-F)-OH, Fmoc-NMe-Ala-OH, Fmoc-D-NMe-Ala, Fmoc-Ala(4-Thz)-OH, Fmoc-NMe-Ala, and chloroacetic acid. After the completion of elongation, the resin was washed with NMP and dichloromethane and then the solvent was distilled off under reduced pressure. TFA:triisopropylsilane:dichloromethane = 5:2.5:92.5 (2 mL) was added thereto, and cleavage and deprotection of the peptide were carried out. After 2 hours, the resin was filtered out and the filtrate was recovered. Further, TFA:triisopropylsilane:dichloromethane = 5:2.5:92.5 (1 mL) was added twice, followed by washing, and the filtrate was recovered. The recovered filtrates were all combined, the solvent was distilled off under reduced pressure, and the residue was thoroughly dried to obtain a crude purified product of a linear peptide. The crude purified product thus obtained was dissolved in a solution of acetonitrile (10 mL) and 0.1 M TEAB buffer:pure water = 1:9 (10 mL), and the solution was adjusted to a pH of 8.5 $\pm$ 0.1. 1 molar equivalent of a TCEP solution (0.5 M) was added thereto, followed by stirring at room temperature for 5 hours. Thereafter, the solvent was distilled off under reduced pressure to obtain a crude purified product of a cyclic peptide. The crude purified product thus obtained was dissolved in a small amount of acetonitrile and purified by reverse phase HPLC (0.1% formic acid aqueous solution/0.1% formic acid-acetonitrile solution) to obtain a cyclic peptide 2 (2.16 mg, yield: 3.7%) as a freeze-dried powder.

MS (ESI m/z): (M+H) 1175.5
RT (min): 1.58

Synthesis of cyclic peptides 3 to 6 and Comparative Examples 1 and 2

Cyclic peptides 3 to 6 were synthesized in the same manner as in the synthesis of the cyclic peptide 1. Comparative Example 1 was synthesized by the method described in WO2021102322. Comparative Example 2 was synthesized by the method described in WO2013123266A.

Table 1 shows the sequences of cyclic peptides. In the table, the cyclic peptide is shown on the left side with an N-terminal chloroacetylated amino acid residue and on the right side with a thiol group-containing compound, and the cyclic peptide is formed by reaction of an N-terminal chloroacetyl group with a thiol group to form a thioether bond. In Comparative Example 1, the left side shows an N-terminal amino acid residue and the right side shows a C-terminal amino acid residue, with both ends of the peptide being linked to each other by an amide bond to form a cyclic peptide. In Comparative Example 2, "Ac" in the table represents an acetyl group, and the amino acid represented as "$r8" in the table is an $\alpha$-MeS8-octenyl-alanine olefin amino acid linked by an all-carbon crosslinker containing one double bond. The amino acid represented as "$" is an $\alpha$MeS5-pentenyl-alanine olefin amino acid linked by an all-carbon crosslinker containing one double bond. In Comparative Example 2, the side chains of amino acid residues represented by "$r8" and "$" are stapled.

[Table 1]

| Cyclic peptide | Sequence | | | | | | | | | |
|---|---|---|---|---|---|---|---|---|---|---|
| 1 | MeAla | Phe(4-F) | Thr | Phe(4-Cl) | MePhe | D-Pro | Hse(Me) | MeAla | C2S | |
| 2 | MeAla | Ala(4-Thz) | D-MeAla | MeAla | Phe(4-F) | Thr | Phe(4-Cl) | MePhe | ProS | |
| 3 | Pro | Phe(4-F) | Thr | Phe(4-Cl) | MePhe | D-MeAla | Hse(Me) | MeAla | C2S | |
| 4 | Pro | Phe(4-F) | Thr | Phe(4-Cl) | MePhe | MeAla | Hse(Me) | MeAla | C2S | |
| 5 | Pro | Phe(4-F) | Thr | Phe(4-Cl) | MePhe | MeAla | Hse(Me) | MeAla | Ala(4-Thz)S | |
| 6 | N(2-MeOEt)Gly | Phe(4-F) | Thr | Phe(4-Cl) | MePhe | MeAla | Hse(Me) | MeAla | Ala(4-Thz)S | |
| Comparative Example 1 | Hse(Me) | MeAla | Ala(4-Thz) | D-MeAla | MeAla | Phe(4-F) | Tle | Phe(4-Cl) | MePhe | D-Pro |
| Comparative Example 2 | Ac-Leu-Thr-Phe-Sr8-Ala-Tyr-Trp-Ala-Gln-Leu-$-Ala-Ala-Ala-Ala-Ala-D-Ala-$NH_2$ | | | | | | | | | |

In Table 1, the following abbreviations have the following meanings.

C2S: a thiol group-containing amino acid analog residue derived from the compound aa3
Ala(4-Thz)S: a thiol group-containing amino acid analog residue derived from the compound aa8
ProS: a thiol group-containing amino acid analog residue derived from the compound aa13

Structure of cyclic peptide

The structure of the cyclic peptide is shown below.

Cyclic peptide 1

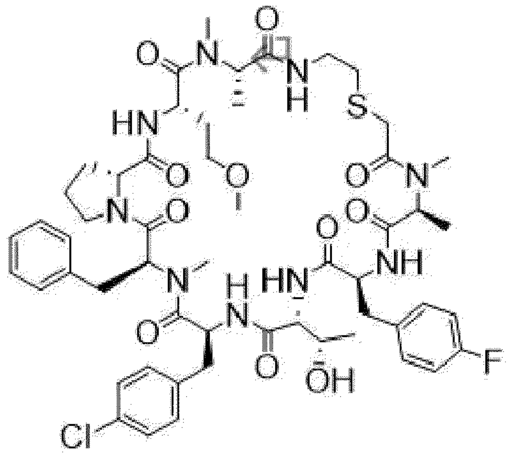

Cyclic Peptide 2

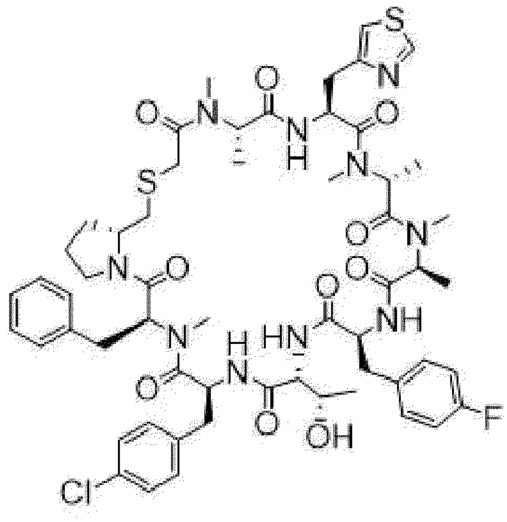

Cyclic peptide 3

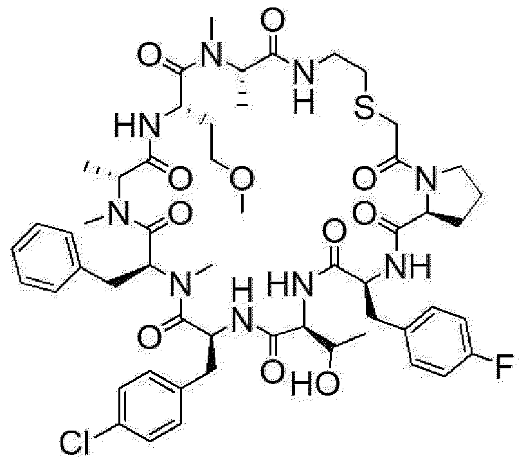

Cyclic peptide 4

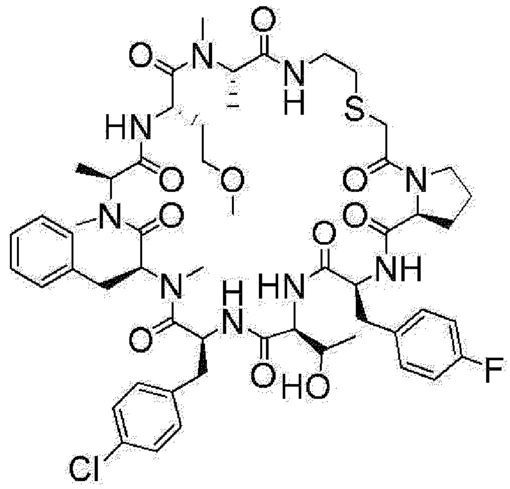

Cyclic peptide 5

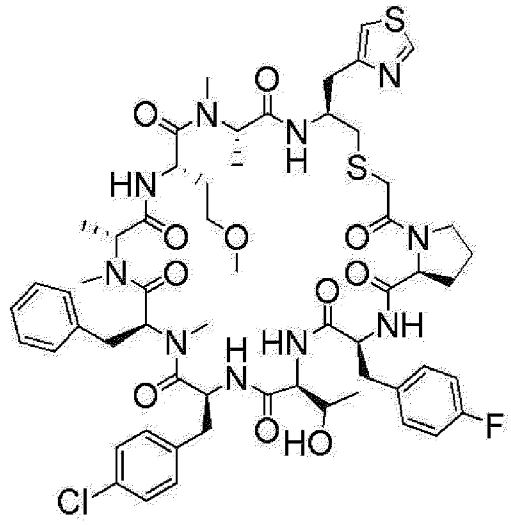

Cyclic peptide 6

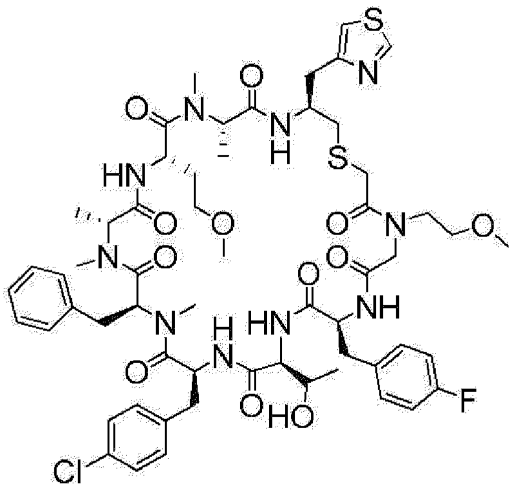

Comparative Example 1

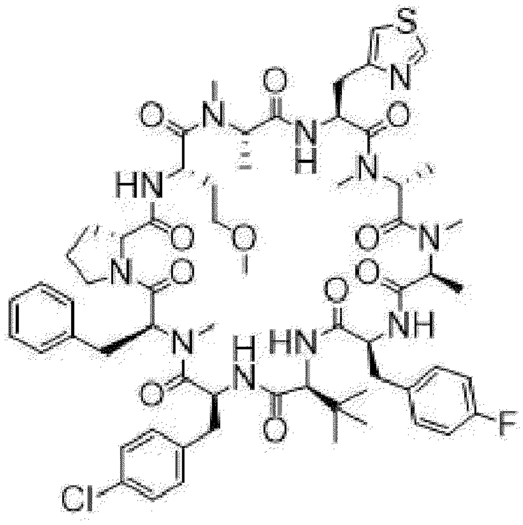

Comparative Example 2

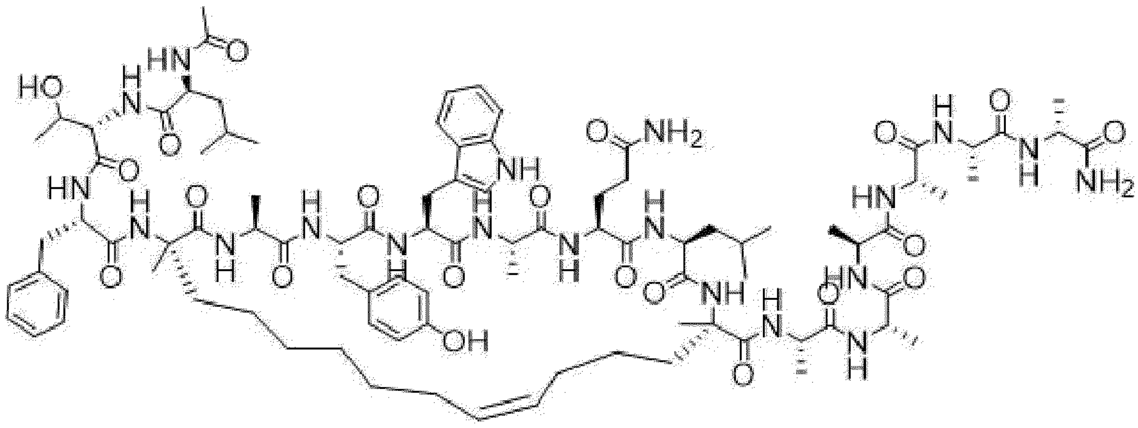

Results of LC-MS measurement

[Table 2]

| Cyclic peptide | Measured value (M+H) | Retention time (min) |
|---|---|---|
| 1 | 1108.5 | 1.54 |
| 2 | 1175.5 | 1.58 |
| 3 | 1108.4 | 1.58 |
| 4 | 1108.5 | 1.54 |
| 5 | 1205.6 | 1.70 |
| 6 | 1223.6 | 1.65 |

<Example 2> Alpha screen assay

The alpha screen assay of the cyclic peptide according to the embodiment of the present invention with respect to MDMX was carried out.

MDMX used was MDMX (24-108, His-tag at the N-terminal; PDB ID: 2MWY, https://www.rcsb.org/structure/2MWY) synthesized by solid phase synthesis. The fragment peptide of p53 [p53(17-26) FITC-labeled, manufactured by AnaSpec, Inc.] was used as a binding partner for MDMX. Donor beads used were Anti-FITC AlphaScreen. Acceptor beads used were Anti-His tag AlphaLISA. The composition of a buffer solution used in the reaction was PBS, 0.1% BSA, and 0.01% Tween 20.

5 μL of a compound having an adjusted concentration (final DMSO concentration: 1% to 5%), 5 μL of MDMX (final concentration: 1 μM), and 5 μL of a p53 peptide (final concentration: 10 nM) were added to each well of a 384-well plate (#6007290, available from PerkinElmer, Inc.) in that order, and then 10 μL of acceptor beads modified with an anti-His tag antibody and 10 μL of donor beads modified with an anti-FITC antibody were added thereto to a final concentration of 20 μg/mL each, and the plate was incubated at room temperature for 1 hour. Finally, the fluorescence signal was measured using Envision. In order to prevent attenuation of a signal due to light in a case of preparing a liquid, an experiment was carried out under a light source equipped with a green filter.

Calculation expression for $IC_{50}$

The fluorescence intensity of each well was normalized by setting the fluorescence intensity of a positive control (a well to which a buffer solution in which the concentration of DMSO was uniform was added instead of adding the compound) as 100% and the fluorescence intensity of a negative control (a well to which a buffer solution in which the concentration of DMSO was uniform was added instead of adding MDMX, p53 peptide, and the compound) as 0%, and $IC_{50}$ was calculated by fitting the normalized fluorescence intensity to a sigmoid curve represented by the following expression.

$$\left(\text{Normalized fluorescence intensity}\right) = 1 - \frac{1}{1 + e^{-a\left[\left(\substack{\text{concentration of}\\ \text{compound}}\right) - IC_{50}\right]}}$$

<Example 3> Evaluation of cell membrane permeability

<Preparation>

Evaluation of the cell membrane permeability of the cyclic peptide according to the embodiment of the present invention was carried out.

300 μL of MDCKII cells (ECACC standard cell line) were seeded at a density of $1.0 \times 10^6$ cells/mL onto an insert (dedicated for a 24-well plate, pore diameter: 3.0 μm, manufactured by Corning Incorporated) and cultured at 37°C in a 5% $CO_2$ environment. After three days, the electric resistance value of the cell layer was measured (using a measuring device), and it was confirmed that the cell layer had high barrier properties (> 100 $\Omega \cdot cm^2$).

<Permeation test>

After the insert was washed by being immersed in HBSS (phenol red-free, the same applies hereinafter), 200 μL of a sample prepared in 10 μM/HBSS was added thereto, and the insert was allowed to stand in a low-adsorption 24-well plate containing 900 μL of HBSS (37°C, 5% $CO_2$). After 2 hours, each liquid of an upper layer (apical) and a lower layer (basal) of the insert (10 μL for apical and 500 μL for basal) was recovered. After testing, no leakage was confirmed with Lucifer Yellow, which is a non-permeable fluorescent dye.

<Quantification>

The device used was LC/MS/MS (triple quadrupole type).

Eluent: A) 5 mM ammonium formate, 0.2% formic acid/$H_2O$, B) 0.1% formic acid/MeCN, flow rate: 0.5 mL/min, injection volume: 2 μL, column: ACQUITY UPLC BEH C18 Column, 1.7 μm, 2.1 mm × 50 mm (manufactured by Waters Corporation), temperature: 70°C, gradient (%B): 2% (0 to 0.5 min) → 98% (2 to 3 min) → 2% (3 to 5 min), ionization: ESI, detection mode: MRM (positive)

Based on the calculation expression shown in Expression (1), a permeability coefficient $P_{app}$, which represents the membrane permeability, was calculated from each quantitative value.

$$P_{app} = V/C0 \times 1/S \times dC/dt \qquad (1)$$

V: basal volume (0.9 mL)
C0: initial concentration (10 $\mu$M)
S: surface area (0.33 $cm^2$) of single layer membrane
dC/dt: concentration change at basal [$\mu$M/s]

<Example 4>

Calculation of molecular shape factor r value using MD calculation

First, a two-dimensionally drawn structural formula of the cyclic peptide is input into Chem3D to create a three-dimensional structure (Fig. 1). Using the present three-dimensional structure as an initial structure, the structure is optimized by a quantum chemical calculation method (B3LYP/6-31G*, software: Gaussian) to obtain a locally stable structure. In the locally stable structure, an electrostatic field generated by the cyclic peptide is obtained by a quantum chemical calculation method (B3LYP/6-31G*, software: Gaussian), and a point charge (RESP charge) is assigned to each atom so as to reproduce the electrostatic field. Next, the state of covalent bonds between the atoms is analyzed (Amber), and van der Waals parameters (gaff2) are assigned to each atom. These charges and van der Waals parameters are collectively referred to as a force field.

Next, under the present force field, using the above locally stable structure as an initial structure, a molecular dynamics (MD) simulation is carried out in chloroform (software: Gromacs and plumed). As an efficient method for efficiently exploring a wide conformation space, the MD simulation employs a replica exchange MD method in which temperatures higher than room temperature are also used in addition to room temperature as temperatures at the time of the simulation. The temperatures used are six types (six types of replicas) and are as shown in the table below. The present temperature is applied only to the cyclic peptide and 298 K is always applied to chloroform present around the cyclic peptide.

[Table 3]

| | Temperature of cyclic peptide/chloroform [K] |
|---|---|
| Lineage 1 | 298/298 |
| Lineage 2 | 348/298 |
| Lineage 3 | 398/298 |
| Lineage 4 | 448/298 |
| Lineage 5 | 498/298 |
| Lineage 6 | 548/298 |

In a case where the calculation for 300 ns was carried out using the present replica exchange MD method, the most stable structure at room temperature was calculated.

For the present most stable structure, the inertia tensor is obtained, the principal moment of inertia is obtained, each axis length (a, b, c) is obtained, and the r value is calculated. Specifically, the r value is calculated according to the following procedure.

The three-dimensional coordinates of the atoms belonging to the main chain of the cyclic peptide having the above most stable structure are represented as ($X_{a,1}, X_{a,2}, X_{a,3}$). Here, a is a label that identifies the atoms belonging to the main chain, and takes an integer from 1 to N. N is the total number of atoms belonging to the main chain of the cyclic peptide.

The r value is calculated for the three-dimensional coordinates. The r value can be calculated according to the following procedure.

(1) Using three-dimensional coordinates as an input, the inertia tensor (a 3 $\times$ 3 matrix) is calculated according to the following expression.

$$\begin{pmatrix} I_{11} & I_{12} & I_{31} \\ I_{21} & I_{22} & I_{32} \\ I_{31} & I_{32} & I_{33} \end{pmatrix} = \begin{pmatrix} \sum_{a=1}^{N}\sum_{k=1}^{3}(X_{a,k})^2 - \sum_{a=1}^{N} X_{a,1}X_{a,1} & -\sum_{a=1}^{N} X_{a,2}X_{a,1} & -\sum_{a=1}^{N} X_{a,3}X_{a,1} \\ -\sum_{a=1}^{N} X_{a,2}X_{a,1} & \sum_{a=1}^{N}\sum_{k=1}^{3}(X_{a,k})^2 - \sum_{a=1}^{N} X_{a,2}X_{a,2} & -\sum_{a=1}^{N} X_{a,3}X_{a,2} \\ -\sum_{a=1}^{N} X_{a,3}X_{a,1} & -\sum_{a=1}^{N} X_{a,2}X_{a,3} & \sum_{a=1}^{N}\sum_{k=1}^{3}(X_{a,k})^2 - \sum_{a=1}^{N} X_{a,3}X_{a,3} \end{pmatrix}$$

(2) Eigenvalues of the inertia tensor are calculated. The obtained three eigenvalues are referred to as principal moments of inertia and are represented by ($I_1$, $I_2$, $I_3$).

(3) As shown in Fig. 2, each of the axis lengths of an ellipsoid with uniform distribution is set to be a, b, and c (a > b > c). Using the principal moments of inertia as an input, each of the axis lengths a, b, and c (a > b > c) of the ellipsoid with uniform distribution is calculated according to the following expression.

$$a = \sqrt{\frac{5}{N}\left[\frac{\sum_{i=1}^{3} I_i}{2} - I_1\right]}$$

$$b = \sqrt{\frac{5}{N}\left[\frac{\sum_{i=1}^{3} I_i}{2} - I_2\right]}$$

$$c = \sqrt{\frac{5}{N}\left[\frac{\sum_{i=1}^{3} I_i}{2} - I_3\right]}$$

(4) Using each of the axis lengths of the ellipsoid as an input, the molecular shape factor (r) is calculated according to the following expression.

$$r = \frac{2\sqrt{b^2 + c^2}}{\sqrt{c^2 + a^2} + \sqrt{a^2 + b^2}}$$

The r value was obtained according to the above-described procedure.

Table 4 summarizes r values and $P_{app}$ of cyclic peptides.

<Evaluation standards for $P_{app}$>

D < 0.1 × $10^{-6}$ cm/sec; 0.1 × $10^{-6}$ cm/sec ≤ C < 0.25 × $10^{-6}$ cm/sec; 0.25 × $10^{-6}$ cm/sec ≤ B < 0.50 × $10^{-6}$ cm/sec; 0.5 × $10^{-6}$ cm/sec ≤ A

The evaluations A, B, and C indicate that the cell membrane permeability is sufficient, and the evaluation D indicates that sufficient cell membrane permeability cannot be obtained.

[Table 4]

| Cyclic peptide | (b) given below | (c) given below | r | $P_{app}$ |
|---|---|---|---|---|
| 1 | Satisfied | Satisfied | 0.46 | C |
| 2 | Satisfied | Satisfied | 0.46 | C |

(continued)

| Cyclic peptide | (b) given below | (c) given below | r | $P_{app}$ |
|---|---|---|---|---|
| 3 | Satisfied | Satisfied | 0.47 | B |
| 4 | Satisfied | Satisfied | 0.43 | C |
| 5 | Satisfied | Satisfied | 0.47 | C |
| 6 | Satisfied | Satisfied | 0.48 | C |
| Comparative Example 1 | Not satisfied | Satisfied | 0.51 | D |
| Comparative Example 2 | Not satisfied | Satisfied | 0.64 | D |
| [0129] (b) The main chain structure of the peptide contains a sulfur atom.<br>(c) The peptide is non-ionic in a physiological environment. | | | | |

[Sequence listing]

International application 22F00771W1JP23030223_34.xml based on International Patent Cooperation Treaty

**Claims**

**1.** A cyclic peptide or a salt thereof, represented by Formula (1) and having the following characteristics (a) to (c),

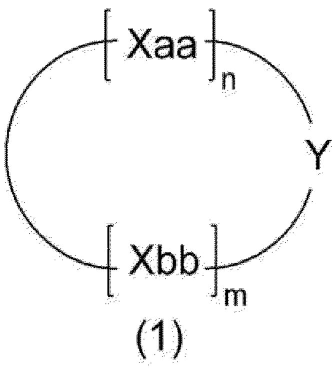

(1)

in the formula, Y represents a divalent group represented by *-CR$^1$-S-CR$^2$-*, $R^1$ and $R^2$ represent a hydrogen atom or a substituent, * represents a position of bonding to Xaa and Xbb,
n pieces of Xaa's each independently represent any amino acid residue or any amino acid analog residue,
m pieces of Xbb's each independently represent any amino acid residue or any amino acid analog residue, and
n + m represents an integer of 5 to 50;

(a) in a structure of the cyclic peptide, in a case where an axis length in a longest axis direction of a main chain structure is denoted by a, and axis lengths in two other directions which are orthogonal to a and are orthogonal to each other are denoted by b and c, a molecular shape factor r calculated by Formula (2) after a step of carrying out an ellipsoidal approximation for obtaining each of the axis lengths a, b, and c is within a range of 0.4 to 0.6;

$$r = \frac{2\sqrt{b^2 + c^2}}{\sqrt{a^2 + b^2} + \sqrt{c^2 + a^2}} \quad (2)$$

(b) the main chain structure of the peptide contains a sulfur atom; and
(c) the peptide is non-ionic in a physiological environment.

**2.** The cyclic peptide or a salt thereof according to claim 1,

wherein the cyclic peptide is represented by Formula (3),

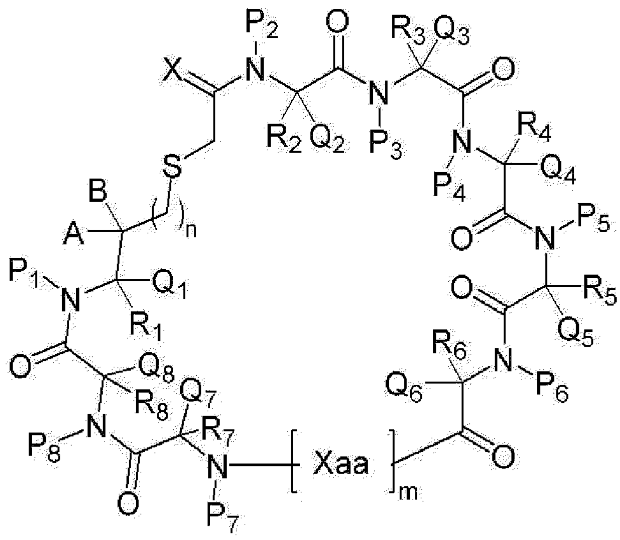

(3)

in the formula, X is an oxygen atom or a sulfur atom,
$R_1$, $R_2$, $R_3$, $R_4$, $R_5$, $R_6$, $R_7$, $R_8$, $Q_1$, $Q_2$, $Q_3$, $Q_4$, $Q_5$, $Q_6$, $Q_7$, $Q_8$, A, and B are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent,
$P_1$, $P_2$, $P_3$, $P_4$, $P_5$, $P_6$, $P_7$, and $P_8$ are each independently a hydrogen atom, an alkyl group which may have a substituent, an alkenyl group which may have a substituent, an alkynyl group which may have a substituent, an aromatic carbocyclic group which may have a substituent, a non-aromatic carbocyclic group which may have a substituent, an aromatic heterocyclic group which may have a substituent, or a non-aromatic heterocyclic group which may have a substituent, or
a carbon atom to which $R_1$, $R_2$, $R_3$, $R_4$, $R_5$, $R_6$, $R_7$, $R_8$, A, and B are bonded may form a heterocycle together with a nitrogen atom to which $P_1$, $P_2$, $P_3$, $P_4$, $P_5$, $P_6$, $P_7$, and $P_8$ are bonded,
m pieces of Xaa's each independently represent any amino acid residue or any amino acid analog residue, and
n is an integer of 0 to 5, and m is an integer of 0 to 2.

**3.** The cyclic peptide or a salt thereof according to claim 2,
wherein n is 0 or 1.

**4.** The cyclic peptide or a salt thereof according to claim 2,
wherein m is 1.

**5.** The cyclic peptide or a salt thereof according to claim 2,
wherein at least one of $R_3$ to $R_8$ or $Q_3$ to $Q_8$ is an aromatic carbocyclic group which may have a substituent or an aromatic heterocyclic group which may have a substituent.

**6.** The cyclic peptide or a salt thereof according to claim 2,
wherein at least one of $R_3$, $R_5$, $R_6$, $R_7$, $R_8$, $Q_3$, $Q_5$, $Q_6$, $Q_7$, or $Q_8$ is an aromatic carbocyclic group which may have a substituent or an aromatic heterocyclic group which may have a substituent.

**7.** The cyclic peptide or a salt thereof according to claim 2,
wherein $R_1$ and $Q_1$ or A and B, and $P_1$ may form a heterocycle together with a carbon atom to which $R_1$ and $Q_1$ or A and B are bonded and a nitrogen atom to which $P_1$ is bonded, a carbon atom to which $R_2$, $R_3$, $R_4$, $R_5$, $R_6$, $R_7$, $R_8$, $Q_2$, $Q_3$, $Q_4$, $Q_5$, $Q_6$, $Q_7$, and $Q_8$ are bonded may form a heterocycle together with a nitrogen atom to which $P_2$, $P_3$, $P_4$, $P_5$, $P_6$, $P_7$, and $P_8$ are bonded, Xaa may be proline, and the cyclic peptide or a salt thereof contains at least one heterocycle by a structure formed from these groups.

**8.** The cyclic peptide or a salt thereof according to claim 2, wherein $R_1$ and $Q_1$ or A and B, and $P_1$ may form a heterocycle together with a carbon atom to which $R_1$ and $Q_1$ or A and B are bonded and a nitrogen atom to which $P_1$ is bonded, a carbon atom to which $R_2$ and $Q_2$ are bonded may form a heterocycle together with a nitrogen atom to which $P_2$ is bonded, Xaa may be proline, and the cyclic peptide or a salt thereof contains at least one heterocycle by a structure formed from these groups.

**9.** The cyclic peptide or a salt thereof according to claim 2, wherein $R_1$ and $Q_1$ or A and B, and $P_1$ may form a pyrrolidine ring together with a carbon atom to which $R_1$ and $Q_1$ or A and B are bonded and a nitrogen atom to which $P_1$ is bonded, a carbon atom to which $R_2$ and $Q_2$ are bonded may form a pyrrolidine ring together with a nitrogen atom to which $P_2$ is bonded, Xaa may be proline, and the cyclic peptide or a salt thereof contains at least one pyrrolidine ring by a structure formed from these groups.

**10.** The cyclic peptide or a salt thereof according to claim 2, wherein $R_1$ and $Q_1$ or A and B and Xaa are each any D-amino acid residue or any D-amino acid analog residue.

**11.** An MDMX inhibitor comprising: the cyclic peptide or a salt thereof according to any one of claims 1 to 10.

FIG. 1

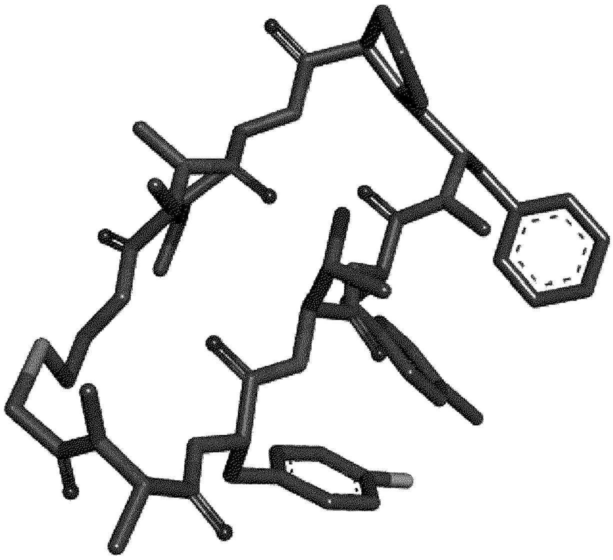

FIG. 2

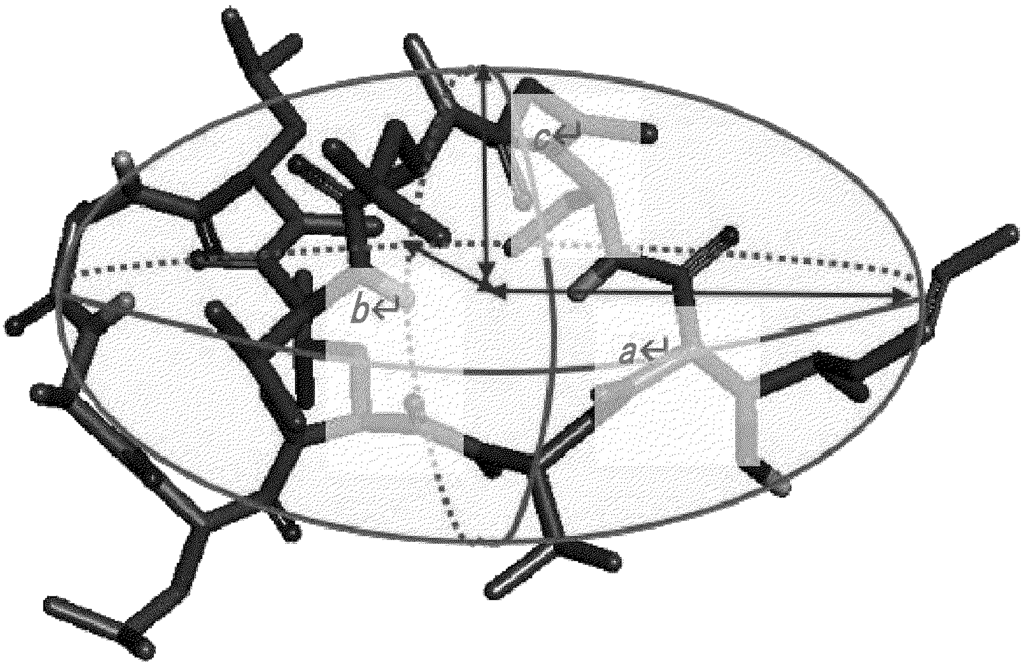

**INTERNATIONAL SEARCH REPORT**

International application No.
**PCT/JP2023/030223**

**A. CLASSIFICATION OF SUBJECT MATTER**

***C07K 4/00***(2006.01)i; ***C07K 7/64***(2006.01)i; ***C12N 15/11***(2006.01)i
FI: C07K7/64; C07K4/00; C12N15/11 Z

According to International Patent Classification (IPC) or to both national classification and IPC

**B. FIELDS SEARCHED**

Minimum documentation searched (classification system followed by classification symbols)

C07K4/00; C07K7/64; C12N15/11

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | US 2022/0002346 A1 (LOKEY, R. S.) 06 January 2022 (2022-01-06) claims, examples | 1-11 |
| A | WO 2021/102322 A1 (UNNATURAL PRODUCTS INC.) 27 May 2021 (2021-05-27) claims, examples | 1-11 |
| A | FURUKAWA, A. et al. Drug-Like Properties in Macrocycles above MW 1000: Backbone Rigidity versus Side-Chain Lipophilicity. Angewandte Chemie, International Edition. 2020, vol. 59, no. 48, pp. 21571-21577, DOI 10.1002/anie.202004550 abstract, fig. 1, 3 | 1-11 |
| A | PYE, C. R. et al. Nonclassical Size Dependence of Permeation Defines Bounds for Passive Adsorption of Large Drug Molecules. Journal of Medicinal Chemistry. 2017, vol. 60, no. 5, pp. 1665-1672, DOI 10.1021/acs.jmedchem.6b01483 abstract, fig. 2 | 1-11 |
| A | FOUCHE, M. et al. Pharmacokinetic Studies around the Mono- and Difunctionalization of a Bioavailable Cyclic Decapeptide Scaffold. ChemMedChem. 2016, vol. 11, no. 10, pp. 1060-1068, DOI 10.1002/cmdc.201600083 abstract, tables 1-9 | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 September 2023** | **17 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/030223

C. DOCUMENTS CONSIDERED TO BE RELEVANT

| Category* | Citation of document, with indication, where appropriate, of the relevant passages | Relevant to claim No. |
|---|---|---|
| A | LAWRENCE, N. et al. Cyclic peptide scaffold with ability to stabilize and deliver a helical cell-impermeable cargo across membranes of cultured cancer cells. RSC Chemical Biology. 2020, vol. 1, pp. 405-420, DOI 10.1039/d0cb00099j<br>abstract, fig. 1, table 1, fig. 3, 4 | 1-11 |
| A | PARTRIDGE, A. W. et al. Incorporation of Putative Helix-Breaking Amino Acids in the Design of Novel Stapled Peptides: Exploring Biophysical and Cellular Permeability Properties. Molecules. 2019, vol. 24, no. 12, p. 2292, DOI 10.3390/molecules24122292<br>abstract, fig. 1, tables 1, 2 | 1-11 |
| A | JP 2015-508777 A (AILERON THERAPEUTICS, INC.) 23 March 2015 (2015-03-23)<br>claims, examples | 1-11 |

Form PCT/ISA/210 (second sheet) (January 2015)

INTERNATIONAL SEARCH REPORT

International application No.
**PCT/JP2023/030223**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

“In the form of an Annex C/ST.25 text file” above should be understood as “in ST. 26 format”.

**INTERNATIONAL SEARCH REPORT**
**Information on patent family members**

International application No.
**PCT/JP2023/030223**

| Patent document cited in search report | Publication date (day/month/year) | Patent family member(s) | Publication date (day/month/year) |
|---|---|---|---|
| US 2022/0002346 A1 | 06 January 2022 | (Family: none) | |
| WO 2021/102322 A1 | 27 May 2021 | JP 2023-502745 A | |
| claims, examples | | US 2022/0411471 A1 | |
| | | EP 4061397 A1 | |
| | | CN 115297883 A | |
| JP 2015-508777 A | 23 March 2015 | US 2013/0210745 A1 | |
| claims, examples | | WO 2013/123267 A1 | |
| | | EP 2819688 A1 | |
| | | CN 104144695 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015153761 A
- WO 2013123266 A
- WO 2021102322 A
- WO 2015030014 A
- WO 2013100132 A
- WO 2008117833 A
- WO 2012074129 A


### Non-patent literature cited in the description


- **R. FASAN** ; **R. L. A. DIAS** ; **K. MOEHLE** ; **O. ZERBE** ; **D. OBRECHT** ; **P. R. E. MITTL** ; **M. G. GRUTTER** ; **J. A. ROBINSON**. Structure-Activity Studies in a Family of β-Hairpin Protein Epitope Mimetic Inhibitors of the p53-HDM2 Protein-Protein Interaction. *ChemBioChem*, 2006, vol. 7, 515-526
- **D. H. YU** ; **Z. Y. XU** ; **S. MO** ; **L. YUAN** ; **X. D. CHENG** ; **J. J. QIN**. Targeting MDMX for Cancer Therapy: Rationale, Strategies, and Challenges. *Front. Oncol.*, 2020, vol. 10 (1389)